# EUROPEAN PATENT APPLICATION

(11) **EP 4 628 045 A1**
(43) Date of publication of application: **08.10.2025**
(21) Application number: 25163219.6
(22) Date of filing: 12.03.2025
(51) Int. Cl.: A61B 90/50

(54) **SURGICAL TECHNIQUES UTILIZING EXTERIOR-FACING DISPLAY OF HEAD-MOUNTED DEVICE**

(30) Priority: 19.03.2024 US 202463567068 P
(71) Applicant: Stryker Corporation, Portage, MI 49002 (US)
(72) Inventor: Herrmann, Florian, 77963 Schwanau (DE); Kumar, Mayank, San Jose, 95148 (US)
(74) Representative: Schott, Jakob Valentin

(57) **Abstract**

A head-mounted device HMD for use in the surgical environment includes an exterior-facing display that is configured to present content. Such content can include human-readable, visual, graphical, or video content. The content can be presented to inform others of surgically relevant content. In some cases, the content includes a trackable graphic that is detectable by a remote camera source, e.g., for tracking the HMD. The content displayed on the exterior-facing display can be dynamically generated or modified based on numerous sources or detected events.

## Description

### CROSS-REFERENCE TO RELATED APPLICATIONS

The subject application claims priority to and all benefits of United States Provisional Application No. 63/567,068, filed March 19, 2024, the entire contents of which are hereby incorporated by reference.

### BACKGROUND

Recently, extended reality has been integrated into the operating room to assist surgeons and staff with the surgical procedure. Typically, the surgeon or staff wear head-mounted display, such as glasses, which present virtual imagery of objects superimposed on the user's real-world view of the surgical site.

To support and guide the surgeon/staff during surgical workflow steps, any surgical information provided to the head-mounted display needs to be transmitted with low latency and within allowed tolerances. Conventional techniques for synchronizing imagery provided to surgical head-mounted displays fail to provide adequate latency monitoring or correction. As a result, there exists a risk that the imagery presented on the surgical head-mounted display is not properly synchronized to the real-world view of the surgical site.

Additionally, to facilitate surgical tracking and navigation using the head-mounted display, the head-mounted display is often tracked by a separate navigation system. Conventional approaches that utilize a separate navigation system require a mechanical tracker be attached to the support structure of the head-mounted display. Such trackers can be bulky and can interfere with the comfort, ergonomics, or functionality of the head-mounted display. The trackers are usually optically based and include either passive (retro-reflective) markers or active (e.g., LED) markers, which reflect/transmit infrared signals to sensors of an optical localizer of the navigation system. When multiple head-mounted displays requiring tracking, each head-mounted displays requires a tracker with a unique arrangement of markers to enable the navigation system to distinguish between trackers. In turn, some trackers require a mechanical support structure uniquely sized for the specific marker arrangement. Although active markers (LEDs) can change their transmission sequence or power, the markers must remain mechanically fixed to the tracker support structure and the head-mounted display.

Such conventional tracking techniques for head-mounted displays have several shortcomings. Firstly, a unique tracker must be created for each different head-mounted displays that requires tracking. Moreover, many trackers, particularly those with optical tracking markers, have parts that must be assembled and separately sterilized. These factors add complexity and cost to the surgical system. Also, such trackers are not intelligently controllable to adapt to the dynamic conditions in the operating room. The tracker configurations are pre-set and cannot adapt or change. For example, conventional markers are fixed to their support structures. As such, the poses (position or orientation), shape, or arrangement of these markers cannot be actively controlled or changed relative to their support structures. If the tracker is placed in a sub-optimal manner, the respective markers will also be sub-optimally placed. If a tracker is rotated relative to the camera, the markers may lose visibility to the camera. As such, conventional trackers for head-mounted displays are quite susceptible to tracking inaccuracies and losing line-of-sight to the camera seeking to track the head-mounted display. Conventional head-mounted display trackers also lack the ability to communicate meaningful information to the navigation system beyond the pose of the head-mounted display. Also, a user has no ability to communicate or interact with such trackers.

Other means of localizing the head-mounted display to the navigation system include introducing a specialized registration device with markers detectable by the head-mounted display and markers detectable by the localizer being fixed to one another on a common structure. However, use of such registration fixtures adds time and cost to the system. Such issues are exacerbated by the use of multiple head-mounted displays and the ever-increasing complexity of tracking systems in the surgical environment.

Moreover, conventional head-mounted displays are user-centric and seriously lack the ability to provide functionality beyond providing an experience for the user of the head-mounted display. For example, conventional head-mounted displays are incapable of externally displaying information (e.g., surgical information, text, graphics, or video) that other people/cameras can observe when remotely viewing the head-mounted display.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided comprising: a head-mounted device (HMD) comprising a controller, an interior-facing display configured to present a virtual object in conjunction with a real-world view, and an exterior-facing display configured to present a computer-generated trackable graphic; and a tracking system comprising a camera being spatially separated from the HMD, the tracking system being configured to detect the trackable graphic with the camera to localize the HMD in a coordinate system of the camera. Also provided are a method of operating the surgical system of the first aspect, the HMD of the first aspect, a method of operating the HMD of the first aspect, and software program products or non-transitory computer readable medium comprising instructions for implementing the first aspect.

According to a second aspect, a surgical system is provided comprising: a head-mounted device (HMD) comprising a controller and an exterior-facing display configured to present a computer-generated trackable graphic; and a tracking system comprising a camera being spatially separated from the HMD, the tracking system being configured to: detect the trackable graphic with the camera; identify a condition signifying a loss of detection of the trackable graphic with the camera; and in response to identification of the condition, transmit a command to the controller of the HMD to modify presentation of the trackable graphic. Also provided are a method of operating the surgical system of the second aspect, the HMD of the second aspect, a method of operating the HMD of the second aspect, and software program products or non-transitory computer readable medium comprising instructions for implementing the second aspect.

According to a third aspect, a surgical system is provided comprising: a head-mounted device (HMD) comprising a controller, an interior-facing display, and an exterior-facing display configured to present a computer-generated trackable graphic; and a tracking system comprising a controller and a camera being spatially separated from the head mounted device, wherein the camera is configured to detect the trackable graphic and obtain metadata associated with the trackable graphic; and wherein the controller of the tracking system is configured to generate content based on the metadata and transmit the content to the controller of the HMD for presentation on one or both of the interior-facing display and the exterior-facing display. Also provided are a method of operating the surgical system of the third aspect, the HMD of the third aspect, a method of operating the HMD of the third aspect, and software program products or non-transitory computer readable medium comprising instructions for implementing the third aspect.

According to a fourth aspect, a surgical system is provided comprising: a head-mounted device (HMD) comprising a controller, an interior-facing display, and an exterior-facing display; and a tracking system being spatially separated from the HMD and comprising a controller configured to transmit metadata to the controller of the HMD; and wherein the controller of the HMD is configured to generate or customize computer-generated content based on the metadata and present the computer-generated content on one or both of the interior-facing display and the exterior-facing display. Also provided are a method of operating the surgical system of the fourth aspect, the HMD of the fourth aspect, a method of operating the HMD of the fourth aspect, and software program products or non-transitory computer readable medium comprising instructions for implementing the fourth aspect.

According to a fifth aspect, a head-mounted device (HMD) is provided for a surgical environment, the HMD comprising: a controller, an interior-facing display; and
an exterior-facing display; wherein the controller is configured to: obtain computer-generated information related to the surgical environment; and present the computer-generated information on the exterior-facing display. Also provided are a method of operating the HMD of the fifth aspect, a surgical system including a tracking system and the HMD of the fifth aspect, a method of operating a surgical system including a tracking system and the HMD of the fifth aspect, and software program products or non-transitory computer readable medium comprising instructions for implementing the HMD of the fifth aspect.

According to a sixth aspect, a surgical system is provided comprising: a head-mounted device (HMD) comprising a controller, an interior-facing display, and an exterior-facing display; and a tracking system comprising a camera being spatially separated from the HMD, and a controller configured to: generate the content, add time stamps to the content with a clock source, and transmit the content with the time stamps to the controller of the HMD for presentation on one or both of the interior-facing display and the exterior-facing display; wherein the controller of the HMD is configured to present a computer-generated trackable graphic on the exterior-facing display, wherein the trackable graphic is dynamically updated and synchronized with the time stamps included with the content; wherein the camera is configured to detect the trackable graphic and obtain the time stamp associated with the trackable graphic at a moment of detection of the trackable graphic; and wherein the controller of the tracking system is configured to compare the obtained time stamp with the clock source to evaluate a latency related to presentation of the content. Also provided are a method of operating the surgical system of the sixth aspect, the HMD of the sixth aspect, a method of operating the HMD of the sixth aspect, and software program products or non-transitory computer readable medium comprising instructions for implementing the sixth aspect.

According to a seventh aspect, a head-mounted device (HMD) is provided comprising: a support structure; and an exterior-facing display supported by the support structure and configured to present a computer-generated trackable graphic to facilitate tracking of a pose of the HMD. Also provided are a method of operating the HMD of the seventh aspect, a surgical system including a tracking system and the HMD of the seventh aspect, a method of operating a surgical system including a tracking system and the HMD of the seventh aspect, and software program products or non-transitory computer readable medium comprising instructions for implementing the HMD of the seventh aspect.

According to an eighth aspect, a head-mounted device (HMD) is provided comprising: a support structure; an exterior-facing display and a controller supported by the support structure, wherein the controller is configured to control the exterior-facing display to generate a computer-generated trackable graphic to facilitate tracking of a pose of the HMD. Also provided are a method of operating the HMD of the eighth aspect, a surgical system including a tracking system and the HMD of the eighth aspect, a method of operating a surgical system including a tracking system and the HMD of the eighth aspect, and software program products or non-transitory computer readable medium comprising instructions for implementing the HMD of the eighth aspect.

According to a ninth aspect, a surgical system is provided comprising: a tracking system; and a head-mounted device (HMD) comprising: an exterior-facing display; and a controller configured to present a computer-generated trackable graphic on the exterior-facing display screen; and wherein the tracking system is configured to detect the computer-generated trackable graphic to facilitate tracking of a pose of the HMD. Also provided are a method of operating the surgical system of the ninth aspect, the HMD of the ninth aspect, a method of operating the HMD of the ninth aspect, and software program products or non-transitory computer readable medium comprising instructions for implementing the ninth aspect.

According to a tenth aspect, a surgical tracking control system is provided comprising: a controller configured to communicate to a head-mounted device (HMD) that includes an exterior-facing display to instruct the HMD to present a computer-generated trackable graphic on the exterior-facing display. Also provided are a method of operating the surgical tracking control system of the tenth aspect, the HMD of the tenth aspect, a method of operating the HMD of the tenth aspect, and software program products or non-transitory computer readable medium comprising instructions for implementing the surgical tracking control system of the tenth aspect.

According to an eleventh aspect, a head-mounted device (HMD) is provided comprising: a support structure; and an exterior-facing display supported by the support structure that is configured to present computer-generated surgical content. Also provided are a method of operating the HMD of the eleventh aspect, and software program products or non-transitory computer readable medium comprising instructions for implementing the HMD of the eleventh aspect.

According to a twelfth aspect, a head-mounted device (HMD) is provided comprising: a support structure; and an exterior-facing display supported by the support structure that is configured to present human-readable surgical content. Also provided are a method of operating the HMD of the twelfth aspect, and software program products or non-transitory computer readable medium comprising instructions for implementing the HMD of the twelfth aspect.

According to a thirteenth aspect, a head-mounted device (HMD) is provided comprising: a support structure; and an exterior-facing display supported by the support structure that is configured to present a video stream related to a surgical procedure. Also provided are a method of operating the HMD of the thirteenth aspect, software program products or non-transitory computer readable medium comprising instructions for implementing the HMD of the thirteenth aspect, and a surgical system including the HMD and a tracking system for providing the video stream of the thirteenth aspect.

According to a fourteenth aspect, a surgical system is provided comprising: a head-mounted device (HMD) comprising an exterior-facing display; and a controller coupled to the HMD, wherein the controller is configured to: detect a condition or event; and generate computer-generated content related to the condition or event for presentation on the exterior-facing display. Also provided are a method of operating the surgical system of the fourteenth aspect, the HMD of the fourteenth aspect, a method of operating the HMD of the fourteenth aspect, and software program products or non-transitory computer readable medium comprising instructions for implementing the fourteenth aspect.

According to a fifteenth aspect, a surgical system is provided comprising: a head-mounted device (HMD) comprising an exterior-facing display and a plurality of physical optical markers; and a controller coupled to the HMD, wherein the controller is configured to present computer-generated content on the exterior-facing display. Also provided are a method of operating the surgical system of the fifteenth aspect, the HMD of the fourteenth aspect, a method of operating the HMD of the fifteenth aspect, and software program products or non-transitory computer readable medium comprising instructions for implementing the fifteenth aspect.

According to a sixteenth aspect, a surgical system is provided comprising: a first head-mounted device (HMD) comprising at least one first exterior-facing display; a second HMD comprising at least one second exterior-facing display; and one or more controller coupled to the first and second HMDs, wherein the one or more controllers are configured to coordinate presentation of computer-generated content on the at least one first and second exterior-facing displays in response to detection of a condition or event. Also provided are a method of operating the surgical system of the sixteenth aspect, the first or second HMD of the sixteenth aspect, a method of operating the first or second HMD of the sixteenth aspect, and software program products or non-transitory computer readable medium comprising instructions for implementing the sixteenth aspect.

According to a seventeenth aspect, a surgical system is provided comprising: a first head-mounted device (HMD) comprising a camera; a second HMD comprising an exterior-facing display configured to present a computer-generated trackable graphic; and one or more controller coupled to the first and second HMDs, wherein the one or more controllers are configured to utilize the camera of the first HMD to detect the computer-generated trackable graphic presented by the second HMD. Also provided are a method of operating the surgical system of the seventeenth aspect, the first or second HMD of the seventeenth aspect, a method of operating the first or second HMD of the seventeenth aspect, and software program products or non-transitory computer readable medium comprising instructions for implementing the seventeenth aspect.

Any of the above aspects can be utilized individually, or in combination.

Any of the above aspects can be utilized individually, or in combination, with any one or more of the following implementations:
Any computer-generated content can be displayed on interior-facing display or the exterior-facing displays of the HMD, including but not limited to: trackable graphics, human readable information, virtual objects, visual content, graphic content, video content, or video stream content. The content can be surgical content. The content can be a bone model, patient imaging data, an implant model, warnings, messages, user ID information, a status indicator, wherein the status indicator conveys one or more of: an operation status of the HMD; a status of a surgical procedure step; a status of a surgical tool operated by a user of the HMD; a status of a tracking system tracking of the HMD, a virtual surgical guide or guidance region configured to assist a user of the HMD in performing a surgical procedure relative to a surgical site, and the like. Content can be obtained from a software application run by equipment in the operating room or can be obtained by any camera source.

The trackable graphic can be a QR code or a dynamic QR code. The trackable graphic can be geometric array of at least three digital fiducials or a point cloud. The HMD can present the trackable graphic with: a specified pose on the exterior-facing display; a specified shape on the exterior-facing display; a specified graphic type on the exterior-facing display; and/or specified display parameters on the exterior-facing display. The HMD can dynamically change parameters of the trackable graphic on the exterior-facing display. Parameters of the trackable graphic can include one or more of: a pose of the trackable graphic; a shape of the trackable graphic; a graphic type of the trackable graphic; a code of the trackable graphic, metadata associated with the trackable graphic, and/or display parameters of the trackable graphic. The HMD can receive information related to an event occurring in the surgical environment and can modify the computer-generated content on the exterior-facing display in response to receiving the information. The HMD can dynamically change parameters of the trackable graphic on the exterior-facing display in response to changes in a relative spatial pose detected between the exterior-facing display and the camera, such as in response to detection of presence or absence of line-of-sight between the camera of the tracking system and the HMD. The HMD is configured to present an alert or notification on the interior-facing display or exterior-facing display related to loss of detectability of the HMD by the tracking system. The HMD can dynamically change parameters of the trackable graphic on the exterior-facing display in response to detected movement of the HMD. The HMD can receive surgical information, and generate the trackable graphic based on the surgical information. The HMD can encode the trackable graphic with time stamps to facilitate synchronization with the camera of the tracking system. The HMD can detect, or receive input signifying, an identify or a type of the tracking system and generate the computer-generated trackable graphic based on the identity or type of tracking system. The trackable graphic can be a first trackable graphic and the HMD can present a second trackable graphic on the exterior-facing display. The second trackable graphic can be for the same or different tracking system than for the first trackable graphic. The second trackable graphic can be the same or of a different configuration than the first trackable graphic. The HMD is configured to continually present the trackable graphic to enable the tracking system to continually detect the trackable graphic with the camera and determine a pose of the HMD in at least five degrees of freedom. Multiple HMDs can be used each including an exterior facing display. The displays can be coordinated with one another. A first HMD can present a first trackable graphic and a second HMD can present a second trackable graphic different from the first trackable graphic. A tracking system can detect the first and second trackable graphics with the camera to localize the first and second HMDs in the coordinate system of the camera. The tracking system can transmit a command to the controller of the first HMD that specifies configuration of the first trackable graphic and transmit a command to the controller of the second HMD that specifies configuration of the second trackable graphic. The HMD is configured to implement a graphical user interface for presentation on one or both of the interior-facing display or the exterior-facing display, wherein the graphical user interface is configured to enable a user to provide input to the controller of the HMD to modify settings or operation of any content presented on the interior-facing display or the exterior-facing display. The tracking system can transmit the virtual object to the controller of the HMD in response to the camera detecting the trackable graphic. The tracking system can be one or more of: another HMD, a surgical navigation system including a localizer, or a tool comprising a camera. As a surgical navigation system, the tracking system can include a camera and at least one tracker detectable by the camera, wherein the at least one tracker is coupled to an object at a surgical site.

The interior-facing display can present content superimposed, overlaid, or combined with real-world views. The content can include any content, including but not limited to: human readable information, virtual objects, visual content, graphic content, video content, or video stream content. The content can be surgical content. The virtual objects can be virtual panels or virtual objects related to surgery. The virtual object can be a virtual surgical guide or guidance region configured to assist a user of the HMD in performing a surgical procedure relative to a surgical site. The virtual surgical guide can include one of: a virtual cutting plane, a virtual target axis, a virtual crosshair target.

The exterior-facing display can: be integrated into the HMD, be removable from and attachable to the HMD, a touch-screen controllable display, and/or be an LED or OLED digital display screen. The exterior-facing display can have a resolution of at least 200 pixels by 200 pixels. The exterior-facing display can be a first exterior-facing display, and the HMD can include a second exterior-facing display arranged to face a different direction from the first exterior-facing display. The exterior-facing display can include a viewing range from 180-degrees to 360-degrees.

The HMD can also support physical active or passive infrared markers that are detectable by the tracking system. The HMD can include a camera, tracking sensor(s), time-of-flight sensor(s), proximity sensor(s), inertial sensor(s), control input sensor(s), transceiver(s), and the like. The camera of the HMD can capture real-world video and the controller of the HMD can combine, in a video stream, the virtual object with the real-world video.

Metadata can be transmitted to the HMD 200 or detected from a trackable graphic presented by the HMD 200. The metadata include user identity information and/or surgical information. The controller of the tracking system is configured generate the metadata based on information obtained by the camera. The tracking system can generate the content to conform to a specific step of the surgical procedure based on the surgical information and can tag the content with the user identity information. The tracking system can generate an update to the content based on the metadata and transmit the updated content to the controller of the HMD for presentation on the interior-facing display and/or exterior-facing display.

Time stamps can be obtained from the HMD or can be transmitted to the HMD. The controller of the tracking system can detect, from obtained time stamps, presence of the latency related to presentation of the content on HMD. The time stamp differences can be compared to a predefined threshold latency. The tracking system can offset the latency by re-synchronizing and re-transmitting the content to the controller of the HMD for presentation on one or both of the interior-facing display and the exterior-facing display. The tracking system can transmit an alert or notification for presentation on one or both of the interior-facing display and the exterior-facing display of the HMD to inform of detected presence of the latency. Any of the above implementations can be performed automatically. Any of the above implementations can be combined in part or in whole with any part of any aspect.

### BRIEF DESCRIPTION OF THE DRAWINGS

Advantages of the present invention will be readily appreciated as the same becomes better understood by reference to the following detailed description when considered in connection with the accompanying drawings.
FIG. 1 is a perspective view of a surgical system, according to one implementation.
FIG. 2 is a schematic view of an example control system can that be used with the surgical system.
FIG. 3 illustrates an example of an HMD that can be used with the surgical system, wherein an interior-facing display and exterior-facing display are shown as enlarged.
Figures 4A-4H illustrate front views of several examples of exterior-facing displays of the HMD presenting content, wherein the examples show what content can be presented, and how such content can be modified, according to several implementations.
Figure 5 illustrates an example first-person view of the interior-facing display of the HMD, wherein a graphical user interface is presented for modifying settings of the HMD, according to one implementation.
Figure 6 is a combined block diagram and flow chart illustrating various information sources and types of information that can influence configuration and/or operation of the HMD and its external-facing display, according to one implementation.
Figures 7A and 7B illustrate interaction between a human observer and the HMD according to two different spatial relationships, wherein the content presented on the external-facing display of the HMD is dynamically modified based on changes in the spatial relation to the human observer, according to one implementation.
Figures 8A-8C illustrate interaction between the HMD and a tracking system, wherein the content presented on the external-facing display of the HMD is dynamically modified based presence/absence of an object obstructing detectability of the HMD by the tracking system, according to one implementation.
Figure 9 illustrates interaction between the HMD and three different types of observers, wherein the content presented on the external-facing display of the HMD is dynamically customized based on the type of observer and the relative spatial relation of the HMD to the respective observer, according to one implementation.
Figure 10A illustrates interaction between two separate HMDs and a tracking system, wherein the content presented on the external-facing display of the HMD is dynamically modified based presence/absence of an object obstructing detectability of the HMD by the tracking system, according to one implementation.
Figure 10B illustrates interaction between two separate HMDs and a tracking system, wherein one HMD detects content presented on the external-facing display of the other HMD based detected presence of an object obstructing detectability of the HMD by the tracking system, according to one implementation.
Figure 11 is a combined system view and sequence diagram illustrating a method involving a tracking system generating/customizing content for presentation on or both of the exterior/interior facing displays of the HMD based on metadata that the tracking system detects from the HMD, according to one implementation.
Figure 12 is a combined system view and sequence diagram illustrating a method involving the HMD presenting/updating content for presentation on or both of the exterior/interior facing displays of the HMD based on metadata that the HMD receives from a tracking system, according to one implementation.
Figure 13 is a combined system view and sequence diagram illustrating a method involving a latency correction technique by which the tracking system and HMD utilize time stamps to detect latency and synchronize content displayed by the HMD, according to one implementation.

### DETAILED DESCRIPTION

### I. EXAMPLE SYSTEM OVERVIEW

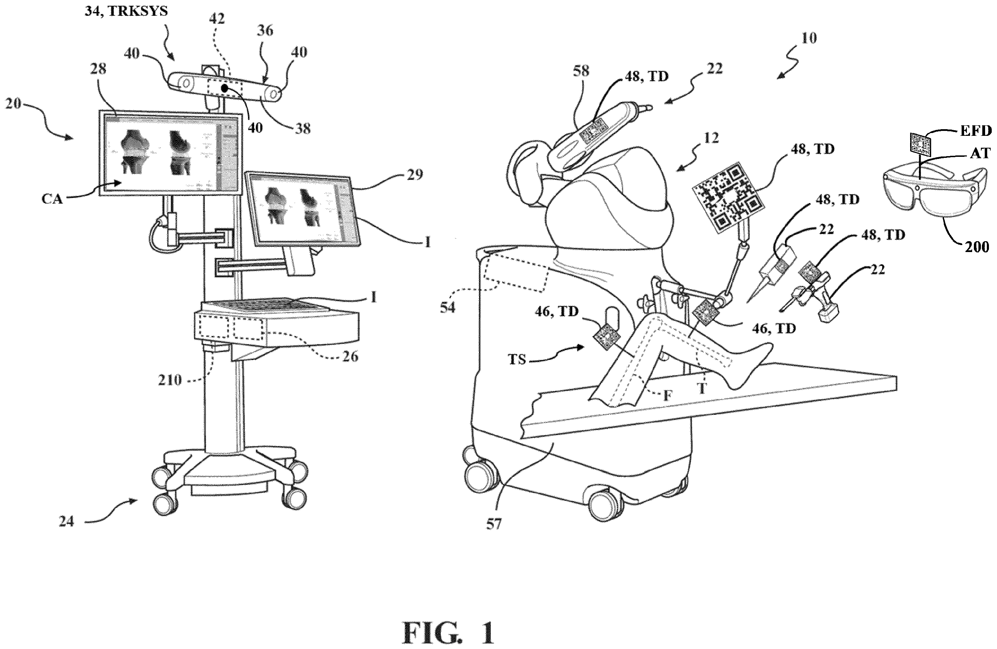

Referring to FIG. 1, a system 10 is provided. The system 10 can be a surgical system adapted for treating a target site TS of a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like. In the example shown in FIG. 1, the patient is undergoing a knee procedure. In addition, the following implementations describe the use of the surgical system 10 for performing a procedure in which material is removed from a femur F and/or a tibia T of a patient. However, it should be recognized that the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical tool is desired.

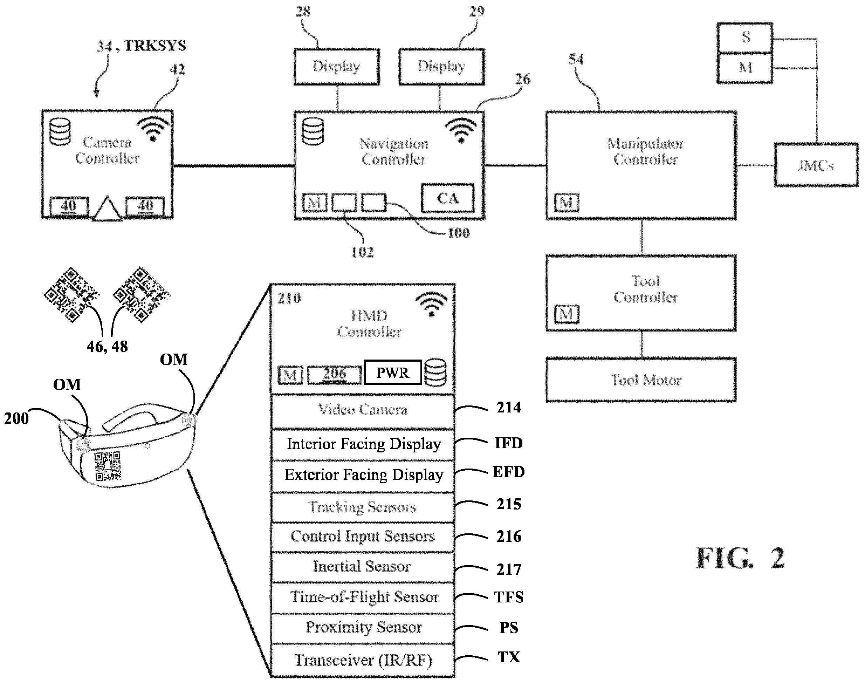

In the implementation shown, the surgical system 10 can include a manipulator 12 (e.g., surgical robot) and a navigation system 20. The navigation system 20 is set up to track movement of various objects in the operating room. Such objects include, for example, any number of surgical tools 22 and the target site TS (e.g., femur F and a tibia T). The navigation system 20 can track these objects for purposes such as displaying their relative positions and orientations to the surgeon on a clinical application (CA) and, in some cases, for purposes of controlling or constraining movement of the surgical tool 22 relative to virtual boundaries (VB) or a surgical plan registered to the target site TS. An example control scheme for the surgical system 10 is shown in FIG. 2.

In the implementation shown, one example of a surgical tool 22 is attached to the manipulator 12. Such an arrangement is shown in U.S. Patent No. 9,119,655, entitled, "Surgical Manipulator Capable of Controlling a Surgical Instrument in Multiple Modes," the disclosure of which is hereby incorporated by reference. In one example, the manipulator 12 has a base 57, a plurality of links 58 extending from the base 57, and a plurality of joints (not numbered) for moving the surgical tool 22 with respect to the base 57. The links 58 and joints form a robotic arm. Some or all of the joints may be passive joints or active joints. The manipulator 12 may have a serial arm or parallel arm configuration. The manipulator 12 can be floor mounted, ceiling mounted, gantry mounted, table mounted, or patient mounted. More than one manipulator 12 can be utilized.

Additionally, or alternatively, the system 10 can include one or more manually operated or hand-held surgical tools 22. For example, the surgical tool 22 may include a hand-held motorized saw, drill, bur, probe, or other suitable tool that may be held and manually operated by a surgeon. Any implementations described with reference to the use of the manipulator 12 may also apply to the use of a hand-held tool 22 with appropriate modifications. The surgical tool 22 may have working end or an energy applicator, such as a rotating bur, saw, router, reamer, impactor, electrical ablation device, cut guide, tool holder, probe, or the like. In other examples, the surgical tool 22 may be a camera tool, such as an endoscope, a laparoscope, an arthroscope, or a microscope. Any of the surgical tools 22 could be supported and moved by the manipulator 12.

The navigation system 20 can include one or more computer cart assemblies 24 that houses one or more navigation controllers 26. A navigation interface is in operative communication with the navigation controller 26. The navigation interface includes one or more displays 28, 29 adjustably mounted to the computer cart assembly 24 or mounted to separate carts as shown. Input devices I such as a keyboard and mouse can be used to input information into the navigation controller 26 or otherwise select/control certain aspects of the navigation controller 26. Other input devices I are contemplated including a touch screen, a microphone for voice-activation input, an optical sensor for gesture input, and the like.

The clinical application CA can be displayed on one or more displays 28, 29 of the navigation system 20. The clinical application CA assists a surgeon or staff in performing the surgical procedure. The clinical application CA can have a plurality of different screens related to the surgical procedure. Such screens can include a pre-operative planning screen, an operating room setup screen, an anatomical registration screen, an intra-operative planning screen, an anatomical preparation screen, or a post-operative evaluation screen, and the like. The clinical application CA can present a navigation guidance region that displays one or more of the surgical objects tracked by a localizer 34 of the navigation system 20.

The localizer 34 communicates with the navigation controller 26. In the implementation shown, the localizer 34 is an optical localizer and includes a camera or camera unit 36. The camera unit 36 has a housing 38 comprising an outer casing that houses one or more optical sensors 40. The optical sensors 40 can detect light signals, such as infrared (IR) signals and/or visible light signals. Camera unit 36 can be mounted on an adjustable arm to position the optical sensors 40 with a field-of-view of the below discussed trackers that, ideally, is free from obstructions. The camera unit 36 includes a camera controller 42 in communication with the optical sensors 40 to receive signals from the optical sensors 40. The camera controller 42 communicates with the navigation controller 26 through either a wired or wireless connection. In other implementations, the optical sensors 40 communicate directly with the navigation controller 26. Position and orientation signals and/or data are transmitted to the navigation controller 26 for purposes of tracking objects. The computer cart assembly 24, display 28, and camera unit 36 may be like those described in U.S. Patent No. 7,725,162 to Malackowski, et al. issued on May 25, 2010, entitled "Surgery System," the disclosure of which is hereby incorporated by reference. The navigation controller 26 can be a personal computer or laptop computer. Navigation controller 26 includes the displays 28, 29, central processing unit (CPU) and/or other processors, memory (not shown), and storage (not shown). The navigation controller 26 is loaded with software that converts the signals received from the camera unit 36 into data representative of the position and orientation of the objects being tracked. The navigation controller 26 includes a navigation processor. It should be understood that the navigation processor could include one or more processors to control operation of the navigation controller 26. The processors can be any type of microprocessor or multi-processor system. The term processor is not intended to limit the scope of any implementation to a single processor.

Navigation system 20 is operable with a plurality of tracking devices 46, 48, also referred to herein as trackers. In the illustrated implementation, one tracker 46 can be firmly affixed to the femur F of the patient and another tracker 46 can be firmly affixed to the tibia T of the patient. Trackers 46 are firmly affixed to sections of bone in an implementation. For example, trackers 46 may be attached to the femur F and tibia T in the manner shown in U.S. Patent No. 7,725,162 to Malackowski, et al. issued on May 25, 2010, entitled "Surgery System," the disclosure of which is hereby incorporated by reference. Trackers 46, 48 may also be mounted like those shown in U.S. Patent Application No. 14/156,856, filed on January 16, 2014, entitled, "Navigation Systems and Methods for Indicating and Reducing Line-of-Sight Errors," hereby incorporated by reference herein. The trackers 46, 48 may be mounted to other tissue types or parts of the anatomy. One or more tool trackers 48 can be coupled to the manipulator 12, the end effector of the manipulator 12, or to the base of the manipulator 12. Tool trackers 48 can also be attached to any of the hand-held tools 22 at any suitable location. Any of these objects can be referred to as surgical tools 22. The tool tracker 48 can be integrated into the surgical tool 22 during manufacture or may be separately mounted to the surgical tool 22 in preparation for surgical procedures.

In one implementation, optical sensors 40 of the localizer 34 receive light signals from the trackers 46, 48. In the example shown, the trackers 46, 48 are implemented as optical-based trackers. For example, the trackers 46, 48 can include a passive detectable tracking object, such as a QR code, which can be detected by the localizer 34. The trackers 46, 48 may include passive markers. For example, the trackers 46, 48 can have at least three passive tracking elements or markers (e.g., reflectors) for transmitting light signals (e.g., reflecting light emitted from the camera unit 36) to the optical sensors 40. In other implementations, some, or all of the trackers 46, 48 may include active tracking markers. The active markers can be, for example, light emitting diodes transmitting light, such as infrared light. Active and passive arrangements are possible. The camera unit 36 receives optical signals from the trackers 46, 48 and outputs to the navigation controller 26 signals relating to the position of the tracking markers of the trackers 46, 48 relative to the localizer 34. Based on the received optical signals, navigation controller 26 generates data indicating the relative positions and orientations of the trackers 46, 48 relative to the localizer 34. These relative positions can be displayed on the clinical application CA as graphical representations for surgical guidance.

Furthermore, in some examples, the navigation system 20 can additionally or alternatively implement the localizer 34 as a vision tracking system. The vision-based localizer 34 includes a vision or video camera coupled to the navigation controller 26. The vision camera can be the one or more of the optical sensors 40. The vision camera facilitates acquisition of 2D and/or 3D machine-vision images or view of structural features that define trackable features such that tracked states of the objects are communicated to (or interpreted by) the navigation controller 26 based on the machine-vision images or view. The machine vision system can be integrated into the camera unit 36, optionally in combination with infrared sensors. The machine vision system can create depth maps and can detect objects with or without trackers. The machine vision system can detect patterns, shapes, colors, computer-codes, tracking geometries, or the like.

Additionally, or alternatively, the navigation system 20 and/or the localizer 34 can employ radio frequency (RF) based tracking. For example, the navigation system 20 may comprise an RF transceiver coupled to the navigation controller 26. Here, the trackers 46, 48 may comprise RF emitters or transponders, which may be passive or may be actively energized. The RF transceiver transmits an RF tracking signal, and the RF emitters respond with RF signals such that tracked states are communicated to (or interpreted by) the navigation controller 26. The RF signals may be of any suitable frequency. The RF transceiver may be positioned at any suitable location to track the objects using RF signals effectively. Furthermore, examples of RF-based navigation systems may have structural configurations that are different than the navigation system 20 illustrated throughout the drawings.

Additionally, or alternatively, the navigation system 20 and/or localizer 34 can employ aspects of electromagnetic (EM) tracking. For example, the navigation system 20 may comprise an EM transceiver coupled to the navigation controller 26. Here, the trackers 46, 48 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation controller 26. The navigation controller 26 may analyze the received EM signals to associate relative states thereto. Examples of EM-based navigation systems may have structural configurations that are different than the navigation system 20 illustrated throughout the drawings.

In other examples, the navigation system 20 and/or the localizer 34 could be based on one or more other types of tracking systems. For example, an ultrasound-based tracking system coupled to the navigation controller 26 could be provided to facilitate acquiring ultrasound images of bone surfaces, landmarks, or markers that define trackable features on the tracked objects such that tracked states are communicated to (or interpreted by) the navigation controller 26 based on the ultrasound images. In other examples, a fluoroscopy-based imaging system (e.g., a C-arm) coupled to the navigation controller 26 could be provided to facilitate acquiring X-ray images of radio-opaque markers that define trackable features such that tracked states are communicated to (or interpreted by) the navigation controller 26 based on the X-ray images. The shape of the trackers 46, 48 can also be of a predetermined geometry that can be identified in X-ray imaging to assist in registration.

Several types of tracking and/or imaging systems could define the localizer 34 and/or form a part of the navigation system 20 without departing from the scope of the present disclosure. Furthermore, the navigation system 20 and/or localizer 34 may have other suitable components or structure not specifically recited herein, and the various techniques, methods, and/or components described herein with respect to the optically-based navigation system 20 shown throughout the drawings may be implemented or provided for any of the other examples of the navigation system 20 described herein. For example, the navigation system 20 may utilize solely inertial tracking and/or combinations of different tracking techniques, sensors, and the like. Any of the described tracking methods can be included in the trackers 46, 48. Other configurations are contemplated.

Based on the position and orientation of the trackers 46, 48 and previously loaded data, navigation controller 26 can determine the position and/or the orientation of the surgical tool 22 relative to the tissue against which the working end is to be applied. In some implementations, the navigation controller 26 forwards these data to a manipulator controller 54. The manipulator controller 54 can then use the data to control the manipulator 12. This control can be like that described in U.S. Patent No. 9,119,655, entitled, "Surgical Manipulator Capable of Controlling a Surgical Instrument in Multiple Modes," or like that described in U.S. Patent No. 8,010,180, entitled, "Haptic Guidance System and Method", the disclosures of which are hereby incorporated by reference.

In one implementation, the manipulator 12 is controlled to stay within a preoperatively defined virtual boundary VB that can be determined by a surgical plan. The virtual boundary VB may be a virtual cutting boundary which defines the material of the anatomy (e.g., the femur F and tibia T) to be removed by the surgical tool 22. For example, each of the femur F and tibia T may have a target volume of material that is to be removed by the working end of the surgical tool 22. The target volumes are defined by one or more virtual cutting boundaries. The virtual cutting boundaries define the surfaces of the bone that should remain after the procedure. The navigation system 20 tracks and controls the surgical tool 22 to ensure that the working end, e.g., the surgical bur, removes the target volume of material and does not extend beyond the virtual cutting boundary, as disclosed in U.S. Patent No. 9,119,655, entitled, "Surgical Manipulator Capable of Controlling a Surgical Instrument in Multiple Modes," the disclosure of which is hereby incorporated by reference, or as disclosed in U.S. Patent No. 8,010,180, entitled, "Haptic Guidance System and Method", the disclosure of which is hereby incorporated by reference.

The virtual cutting boundary VB may be defined within a virtual model of the anatomy (e.g., the femur F and tibia T), or separately from the virtual model. The virtual cutting boundary may be represented as a mesh surface, constructive solid geometry (CSG), voxels, or using other boundary representation techniques. The surgical tool 22 may be used to cut away material from the femur F and tibia T to receive an implant. The surgical implants may include unicompartmental, bicompartmental, or total knee implants as shown in U.S. Patent No. 9,381,085, entitled, "Prosthetic Implant and Method of Implantation," the disclosure of which is hereby incorporated by reference. Other implants, such as hip implants, shoulder implants, spine implants, and the like are also contemplated. The focus of the description on knee implants is provided as one example. These concepts can be equally applied to other types of surgical procedures, including those performed without placing implants.

The navigation controller 26 also generates image signals that indicate the relative position of the working end to the tissue. These images can be presented on the displays 28, 29 by the clinical application CA to allow the surgeon and staff to view the relative position of the working end to the target site TS.

Prior to the start of the intraoperative procedure, preoperative images of the femur F and tibia T may be generated (or of other portions of the anatomy in other implementations). The preoperative images can be stored as two-dimensional or three-dimensional patient image data in a computer-readable storage device, such as memory (M) within the navigation system 20. The patient image data may be based on X-ray scans or computed tomography (CT) scans of the patient's anatomy. The patient image data may then be used to generate two-dimensional images or three-dimensional models of the patient's anatomy. The pre-operative data and models may be used for purposes of surgical planning purposes and intraoperative guidance. For example, the surgical plan (e.g., tool path or resection volume or boundaries VB), may be planned relative to the virtual model. The virtual model and surgical plan can then be registered to the anatomy using any appropriate registration technique, such as pointer registration, imageless registration, or the like.

FIG. 2 illustrates a schematic view of an example control system that be used with the surgical system 10. A localization engine 100 is a software module that can be considered part of the navigation system 20. Components of the localization engine 100 run on navigation controller 26. In some implementations, the localization engine 100 may run on the manipulator controller 54. Localization engine 100 receives as inputs the signals from the localizer 34 and, in some implementations, signals from the trackers 46, 48. Based on these signals, localization engine 100 can determine the pose of each tracker coordinate system in the localizer coordinate system. The localization engine 100 forwards the signals representative of the poses of trackers 46, 48 to a coordinate transformer 102. Coordinate transformer 102 is a navigation system software module that runs on navigation controller 26. Coordinate transformer 102 references the data that defines the relationship between the preoperative images of the patient and the anatomy trackers 46. Coordinate transformer 102 can also store the data indicating the pose of the working end of the surgical tool 22 relative to a tool tracker 48. During the procedure, the coordinate transformer 102 receives the data indicating the relative poses of the trackers 46, 48 to the localizer 34. Based on these data, the previously loaded data, and encoder data from the manipulator 12, the coordinate transformer 102 generates data indicating the position and orientation of the working end of the surgical tool 22 relative to the tissue (e.g., bone) against which the working end is applied. Image signals representative of these data are forwarded to displays 28, 29 enabling the surgeon and staff to view this information. In certain implementations, other signals representative of these data can be forwarded to the manipulator controller 54 to guide the manipulator 12 and corresponding movement of the surgical tool 22.

The manipulator 12 has the ability to operate in a manual mode or a semi-autonomous mode in which the surgical tool 22 is moved along a predefined tool path, as described in U.S. Patent No. 9,119,655, entitled, "Surgical Manipulator Capable of Controlling a Surgical Instrument in Multiple Modes," the disclosure of which is hereby incorporated by reference, or the manipulator 12 may be configured to move in the manner described in U.S. Patent No. 8,010,180, entitled, "Haptic Guidance System and Method", the disclosure of which is hereby incorporated by reference.

The manipulator controller 54 can use the position and orientation data of the surgical tool 22 and the patient's anatomy to control the manipulator 12 as described in U.S. Patent No. 9,119,655, entitled, "Surgical Manipulator Capable of Controlling a Surgical Instrument in Multiple Modes," the disclosure of which is hereby incorporated by reference, or to control the manipulator 12 as described in U.S. Patent No. 8,010,180, entitled, "Haptic Guidance System and Method", the disclosure of which is hereby incorporated by reference.

The manipulator controller 54 may have a central processing unit (CPU) and/or other manipulator processors, memory, and storage. The manipulator controller 54, also referred to as a manipulator computer, is loaded with software as described below. The manipulator processors could include one or more processors to control operation of the manipulator 12. The processors can be any type of microprocessor or multi-processor system. The term processor is not intended to limit any implementation to a single processor.

Position sensors S can be associated with the plurality of links 58 of the manipulator 12. In one implementation, the position sensors S are encoders. The position sensors S may be any suitable type of encoder, such as rotary encoders. Position sensors S can be associated with a joint actuator, such as a joint motor M. The position sensor S is a sensor that monitors the angular position of one of six motor driven links 58 of the manipulator 12 with which the position sensor S is associated. Multiple position sensors S may be associated with each joint of the manipulator 12 in some implementations. The manipulator 12 can also include a force/torque sensor coupled between the distal end of the manipulator 12 and the end effector for detecting manual forces/torques exerted on the tool 22 by an operator. The input forces/torques can be used to command movement of the manipulator 12 and/or to detect collisions with the tool 22.

In some modes, the manipulator controller 54 determines the desired location to which the surgical tool 22 should be moved. Based on this determination, and information relating to the current location (e.g., pose) of the surgical tool 22, the manipulator controller 54 determines the extent to which each of the plurality of links 58 needs to be moved in order to reposition the surgical tool 22 from the current location to the desired location. The data regarding where the plurality of links 58 are to be positioned is forwarded to joint motor controllers JMCs that control the joints of the manipulator 12 to move the plurality of links 58 and thereby move the surgical tool 22 from the current location to the desired location. In other modes, the manipulator 12 is capable of being manipulated as described in U.S. Patent No. 8,010,180, entitled, "Haptic Guidance System and Method", the disclosure of which is hereby incorporated by reference, in which case the actuators are controlled by the manipulator controller 54 to provide gravity compensation to prevent the surgical tool 22 from lowering due to gravity and/or to activate in response to a user attempting to place the working end of the surgical tool 22 beyond a virtual boundary VB.

To determine the current location of the surgical tool 22, data from the position sensors S is used to determine measured joint angles. The measured joint angles of the joints are forwarded to a forward kinematics module, as known in the art. Based on the measured joint angles and preloaded data, the forward kinematics module determines the pose of the surgical tool 22 in a manipulator coordinate system. The preloaded data are data that define the geometry of the plurality of links 58 and joints. With this encoder-based data, the manipulator controller 54 and/or navigation controller 26 can transform coordinates from the localizer coordinate system into the manipulator coordinate system, vice versa, or can transform coordinates from one coordinate system into any other coordinate system described herein using transformation techniques. In many cases, the coordinates of interest associated with the surgical tool 22 (e.g., the tool center point or TCP), the virtual boundaries, and the tissue being treated, are transformed into a common coordinate system for purposes of relative tracking and display.

While the example surgical system 10 has been described with reference to the Figures, the surgical system 10 is not intended to be limited to what is specifically shown and described. For example, the surgical system 10 may not include the manipulator 12 or the navigation system 20 as specifically shown. Other systems are contemplated without departing from the scope of the disclosure.

### II. HEAD MOUNTED DEVICE

One or more head-mounted devices (HMDs) 200 may be incorporated into the surgical system 10. The HMD 200 may be employed to enhance visualization before, during, and/or after surgery. The HMD 200 is an extended reality device, which can include aspects of augmented reality, mixed reality, virtual reality, and the like. The HMD 200 can be used to visualize any surgical information, such as virtual objects, features, instructions, warnings, etc. Any information previously described as being visualized on the displays 28, 29 of the navigation system can be displayed on the HMD 200. The HMD 200 can be used to assist with visualization of the volume of material to be cut from the patient, to help visualize the size of implants and/or to place implants for the patient, to assist with registration and calibration of objects being tracked via the navigation system 20, to see instructions and/or warnings, among other uses, as described further below.

During use, for example, the localizer 34 and/or the navigation controller 26 can send data on an object (e.g., the cut volume model) to the HMD 200 so that the HMD 200 knows where the object is in the HMD coordinate system and can display an appropriate content in the HMD coordinate system. Once registration is complete, then the HMD 200 can be used to visualize computer-generated content in desired locations with respect to any objects in the operating room. Although certain transforms have been described, it is understood that the HMD 200 can operate without requiring any such transforms. The HMD 200 can display content without registering to the bone, or any part of the surgical system 10.

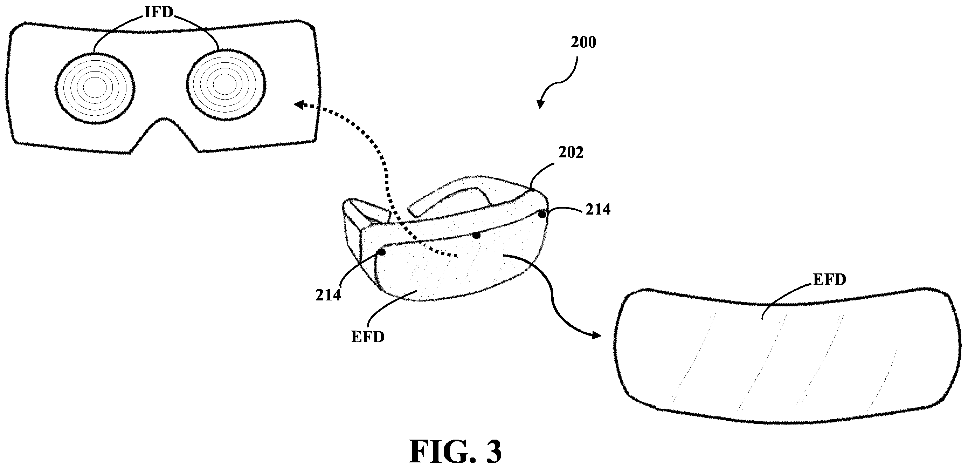

As shown in FIG. 3, the HMD 200 includes a support structure 202, which may be head-mountable in the form of an eyeglass or glasses, headwear or headset, or eyewear (such as a digital contact lens or lenses). The HMD 200 may include additional headbands or supports to hold the HMD 200 on the user's head. In other implementations, the HMD 200 may be integrated into a surgical helmet or other structure worn on the user's head, neck, and/or shoulders. Although not shown, it is contemplated that instead of the HMD 200, an extended reality display screen, such as a monitor, tablet, or hand-held display may be used, which can include similar hardware and capabilities as the described HMD 200.

### 1. Interior Facing Display

As shown in FIGS. 2, 3, 5, and 11-13, the HMD 200 includes one or more interior-facing displays IFD to present content specifically for the wearer of the HMD 200. The interior-facing display IFD faces the eyes of the wearer, and hence, can also be referred to as the HMD user-facing display. The location of the interior-facing display IFD relative to the HMD 200 is shown with a dashed arrow, for illustrative purposes. On the interior-facing display IFD, computer-generated content can be displayed onto a real-world view. The interior-facing display IFD can present computer-generated content combined with the real-world view or a holographic/superimposed/overlay of computer-generated content over the real-world view. In one example, the real-world view is acquired by a video camera 214 attached to the HMD. The video camera 214 produces a live video stream of the real-world and the computer-generated content may be combined into video stream of the real world. The video camera 214 can be located on a front face of the HMD 200, as shown in FIG. 3, for example. In such instances, the interior-facing display IFD may include one or more high-resolution displays positioned in front of the user's eyes.

The interior-facing display IFD may be opaque in such scenarios such that the user cannot naturally see through the interior-facing display IFD. The interior-facing display IFD may include one display for both eyes or a separate display for each eye (as shown in FIG. 3). The interior-facing display IFD can by any type of digital display, such as any type of LED, LCD, OLED, micro-OLED, Quantum LED (QNED, QLED), micro-LED, mini-LED, dot matrix LED, RGB display, or the like.

In other implementations, the HMD 200 may implement natural see-through techniques whereby the HMD interior-facing display IFD is implemented as a transparent lens/visor/waveguide provided between the user's eyes and the real-world. The real-world view is acquired naturally by the user's eyes and the computer-generated content is provided on the transparent lens/visor/waveguide. Such see-through techniques can include a diffractive waveguide, holographic waveguide, polarized waveguide, reflective waveguide, or switchable waveguide.

### i. Display of Virtual Objects on Interior-Facing Display

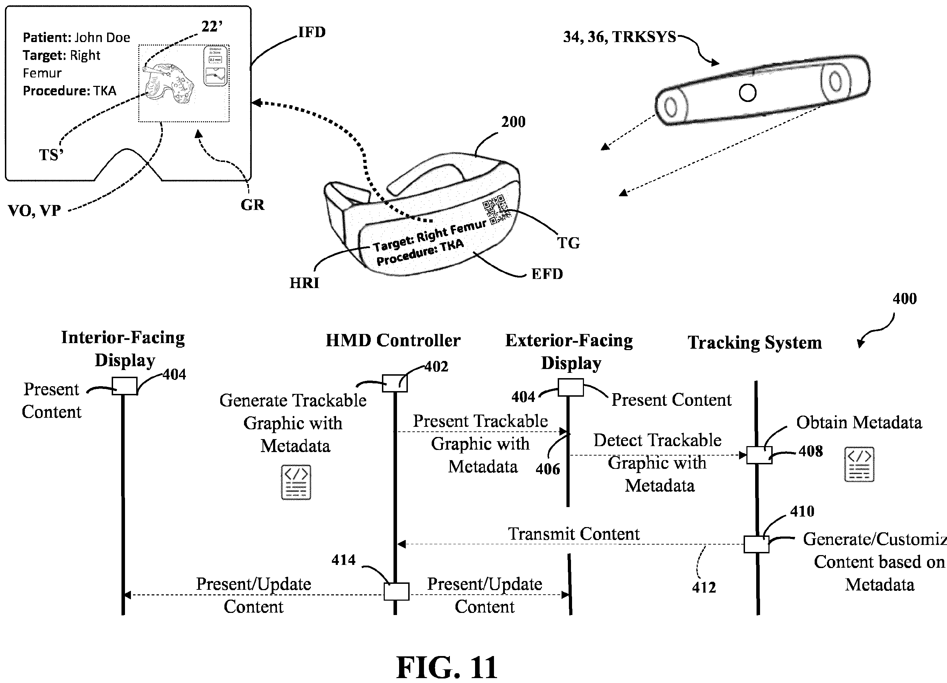

As shown in FIGS. 11-13, the interior-facing display(s) IFD is configured to present one or more virtual object(s) VO onto a real-world view. The virtual object(s) VO are computer-generated text, graphics, or video and can be any content related or relevant to the surgeon, patient, or surgical procedure. The surgical content may, but need not, be related to the process of actually performing surgery. The surgical content can be pre-operative, intraoperative, or post-operative surgical information. Examples of surgical content include but are not limited to: patient information, medical images (e.g., CT scan or volume, X-rays, etc.), surgical guidance information (e.g., tool interaction with target site), surgical planning information, an anatomical model, an implant model, a cut plan, a resection plan or volume, a virtual boundary VB or cutting boundary, surgical tool information, operating room or tool setup information, surgical step information, clinical application information, surgical alerts, notifications or warnings, and the like. The surgical content can be a step of the surgical procedure. The step of the surgical procedure can include but are not limited to: a pre-operative planning step, an operating room setup step, an anatomical registration step, an intra-operative planning step, an anatomical preparation step, or a post-operative evaluation step. The virtual object(s) VO can be a 3D and/or 2D surgical object relevant to any of the above information. Examples of such virtual objects VO include but are not limited to: virtual screens, windows, or information panels, a 3D model of a bone, a 3D model of an implant, and a 3D surgical plan.

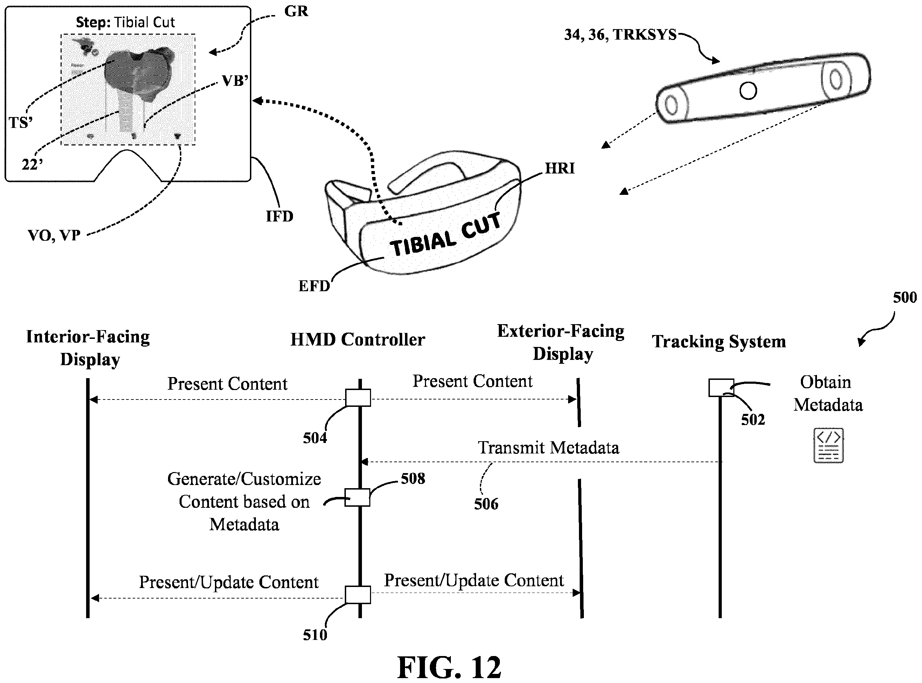

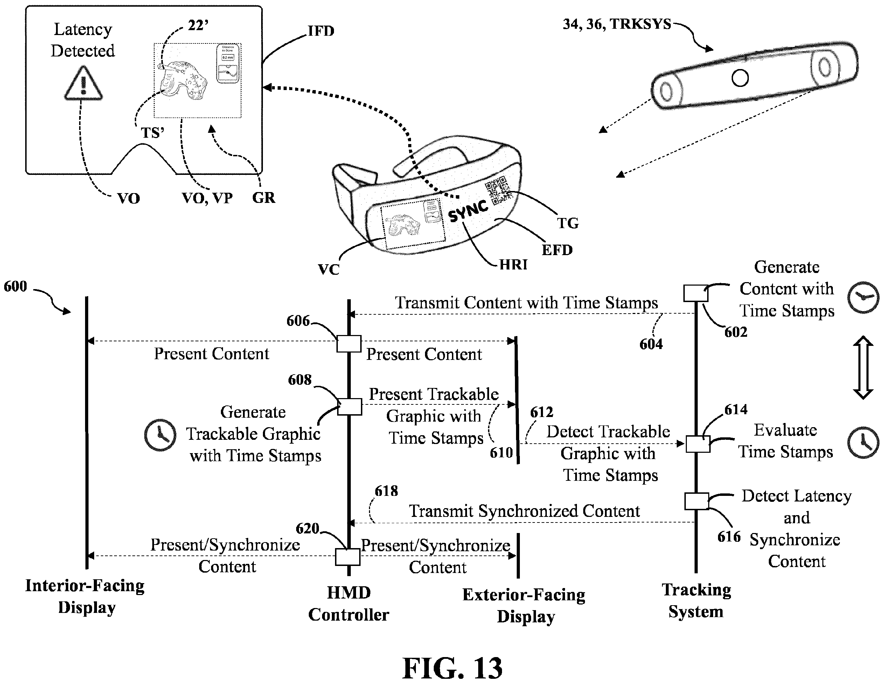

In one example, the virtual object(s) VO can be a virtual panel VP, such as those shown in FIGS. 11-13. The virtual panel VP can include 2D and/or 3D graphical elements, as well as text. The virtual information panel VP can be configured to present any relevant information in a "window" style format. The virtual panel VP can have minimal thickness to emulate a flat object, such as a flat screen. In FIG. 11, the virtual object VO presented on the interior-facing display IFD includes a virtual panel VP that presents a video stream VS of a guidance region GR obtained from the clinical application CA of the navigation system 20. The guidance region GR in FIG. 11 relates to bone registration and shows a real time relationship between a graphical representation of a pointer tool 22' and a graphical representation of the target site TS'. Other text related to the patient, target site and procedure are presented on interior-facing display(s) IFD. In FIG. 12, the virtual object VO presented on the interior-facing display IFD similarly includes a virtual panel VP that presents a video stream of a guidance region GR obtained from the clinical application CA of the navigation system 20. The guidance region GR in FIG. 12 relates to bone preparation and shows a real time relationship between a graphical representation of a cutting tool 22', a graphical representation of the target site TS', and a virtual boundary VB'. Other text related to the particular step of procedure is presented on interior-facing display(s) IFD. In FIG. 13, the virtual object VO presented on the interior-facing display(s) IFD includes a bone model of the target site, as well as graphical icon and text related to an alert (e.g., latency detected). Numerous other examples are contemplated regarding the presentation of virtual objects VO or content on the interior-facing display(s) IFD. Examples of when, where, how, and when virtual objects VO or content can be presented on the interior-facing display(s) IFD of the HMD 200 can be like that those described in U.S. Provisional Patent Application No. 63/551719, filed February 9, 2024, entitled "Extended Reality Systems And Methods For Surgical Applications", the entire contents of which are hereby incorporated by reference.

In other examples, the virtual object(s) VO can any type of virtual surgical guide configured to assist a user of the HMD 200 in performing a surgical procedure relative to a surgical site. The virtual surgical guide may include any of: a virtual cutting plane, a virtual target axis, a virtual crosshair target. Such guides could be derived from the video stream VS of the clinical application CA or could be generated/customized by the tracking system TRKSYS, navigation system 20, HMD 200, or any other described controller.

### 2. Exterior-Facing Display

As shown throughout the Figures, the HMD 200 includes one or more exterior-facing displays EFD for externally displaying computer-generated content (e.g., surgical information, text, graphics, or video) that people/machines (e.g., other than the wearer of the HMD) can observe when remotely viewing the HMD 200. Here, "exterior facing" means the display is faced to be visible to any person/camera (e.g., in the operating room) observing the display from a pose that is spaced apart from and external to the HMD 200. The various uses of the exterior-facing displays EFD will be described in the subsequent section. The exterior-facing display EFD can be supported by the support structure 202 of the HMD 200. In one example, as shown throughout the figures, the exterior-facing display EFD is located on the front face of the HMD 200 and placed in front of the interior-facing display IFD relative to the support structure 202. In other implementations, the exterior-facing display EFD may be located on or more sides of the HMD 200, extending above the HMD 200, or anywhere else relative to the support structure 202. The exterior-facing display EFD may be opaque so that neither the HMD 200 wearer nor other people/cameras can see through the exterior-facing display EFD. In other examples, the exterior-facing display EFD can be transparent or semi-transparent, enabling the HMD 200 wearer and other people/cameras to see through the exterior-facing display EFD.

The exterior-facing display EFD may include one or more high-resolution digital displays. The exterior-facing display EFD can by any type of digital display, such as any type of LED, LCD, OLED, micro-OLED, Quantum LED (QNED, QLED), micro-LED, mini-LED, dot matrix LED, RGB display, or the like. The exterior-facing display EFD can have any resolution sufficient to meaningfully convey digital content and/or graphics. In one example, the exterior-facing display EFD has a resolution of at least 200 pixels by 200 pixels, and up to 4K or higher resolution. The exterior-facing display EFD can be a touch-screen controllable display such that a user can interact and control aspects of the exterior-facing display EFD or HMD 200. For example, the touch screen can be enabled by capacitive or infrared-based technology and can be a layer of the exterior-facing display EFD.

The exterior-facing display EFD shown in the figures are provided only as example implementations. The exterior-facing display EFD may have various sizes, shapes, and configurations as described herein, and should not be limited to the examples shown. In some examples, such as those shown, the exterior-facing display EFD can have a curved or contoured configuration. In other examples, the exterior-facing display EFD can have a flat, irregular, and/or rounded configuration. The exterior-facing display EFD can have other types of configurations, including but not limited to a cube shape, semi or partially spherical configuration, a semi or partially cylindrical configuration, or the like. For example, the exterior-facing display EFD can be a continuous cylindrical screen that surrounds the HMD 200.

The exterior-facing display EFD can include any suitable shape and size. The exterior-facing display EFD may occupy all, substantially all, or part of any one or more faces of the HMD 200. In some instances, the HMD 200 can include multiple exterior-facing displays EFD that can face in different directions from one another. For example, the support structure 202 can support a separate exterior-facing display EFD on different sides of the HMD 200. If the exterior-facing display EFD has a 3D geometry, a separate exterior-facing display EFD can be provided on each face of the 3D geometry. Such multi-screen configurations can be implemented for any 3D geometric shape of the exterior-facing display EFD. For instance, a flat exterior-facing display EFD can also include an exterior-facing display EFD on the opposing backside. When the exterior-facing display EFD is a (partial or full) sphere or cylinder or curved surface, the exterior-facing display EFD can span up to 360 degrees to provide a viewing angle from any perspective. Also, the exterior-facing display EFD could itself, form part of the support structure 202 of the HMD 200.

In one example, as shown in FIG. 3 for instance, the exterior-facing display EFD is integrated (permanently) as part of the HMD 200. Such integration can be at the front-face of the HMD 200 or extending from the body of the HMD 200, such as extending above or to the side of the HMD 200.

In another implementation, to provide more versatility, the exterior-facing display EFD can be releasably attached to and removed from the HMD 200. For example, the exterior-facing display EFD can have a compact configuration and may be easily grasped by the hand of a user to attach to the HMD 200. With such configurations, the exterior-facing display EFD may be released from the HMD 200 and used for other objects and purposes, such as attaching to another surgical object requiring tracking. The exterior-facing display EFD can be attached to the HMD 200 in preparation for surgery and removed from the HMD 200 after use. With such attachable configurations, the exterior-facing display EFD can have any suitable dimension. For example, the exterior-facing display EFD can have a volume (length, width, depth) of 50x50x10mm. The dimension of the exterior-facing display EFD may be sized as needed for the appropriate application.

As shown in FIG. 1, for example, the exterior-facing display EFD can be releasably attached to the HMD 200 using an attachment AT. The attachment AT can be a holder attached to or extending from the support structure 202. The exterior-facing display EFD can be clamped, pinned, mounted, fastened, or secured to the HMD 200 in any manner. For example, the exterior-facing display EFD could be secured to an elastic band, bendable strip that can be secured around the HMD 200. The attachment AT can be a kinematic attachment like that described in US. Patent No. 10,537, 395, entitled "Navigation Tracker with Kinematic Connector Assembly", the entire contents of which are hereby incorporated by reference. Any components of the attachment AT can be directly coupled to the support structure 202 of the HMD 200.

In another example, the exterior-facing display EFD is a portable electronic device (such as a smartphone, tablet, etc.) that has capabilities beyond being used as the exterior-facing display EFD of the HMD 200. Such supplemental capabilities include personal use, such as the capability for cellular communication, storing photos, or any other feature provided by smartphones and tablets. In some cases, the portable electronic device can be a folding device, such as a paper-thin folding device. When the exterior-facing display EFD is a portable electronic device, the attachment AT can be a rigid or adjustable device holder sized for clamping to or slidably receiving the portable electronic device.

Other example means of attaching the exterior-facing display EFD to the HMD 200 can be like those described in: US Patent App. Pub. No. 2023/0338093, entitled "Sterilizable Surgical Device With Battery Switch", US Patent App. Pub. No. 2022/0257334, entitled "Clamp Assembly For Fixing A Navigation Tracker To A Portion Of Bone", US Patent App. No. 18/392,014, entitled "Surgical Clamp Assembly For Fixing A Navigation Tracker To A Portion Of Bone", US Provisional Patent App. No. 63/526,733, entitled "Surgical Tracker Assemblies With Optimized Size, Weight, And Accuracy", and/or US Provisional Patent App. No. 63/472138, entitled "Assembly And Method For Mounting A Tracker To Bone", the entire contents of these disclosures being hereby incorporated by reference.

In situations where the exterior-facing display EFD can be removed from the HMD 200, it is contemplated that the exterior-facing display EFD can be sterilizable and configured to be used in the sterile field. The exterior-facing display EFD may be hermetically sealed, or placed under a sealed transparent layer that is also hermetically sealed and thermally protected. The exterior-facing display EFD can be removed from the HMD 200 to facilitate sterilization. In one example, the exterior-facing display EFD can have a housing including a cavity disposed therein for a controller or PCB. The cavity can open to the exterior of the EFD housing through at least one channel formed in the housing. The PCB can be coated with a heat-resistive coating, such as a Parylene coating. The exterior-facing display EFD can be sterilized and the sterilization fluid can enter the cavity and drain from the channels during sterilization. To facilitate this capability, the exterior-facing display EFD can employ the configuration and features of those described in: US Patent App. Pub. No. 2023/0338093, entitled "Sterilizable Surgical Device With Battery Switch", the entire contents of which are hereby incorporated by reference.

In another example, the exterior-facing display EFD is configured to interface with a surgical or sterile drape that prevents exposure of the exterior-facing display EFD with the sterile field. In one example, the drape can include a transparent window configured to cover the exterior-facing display EFD while enabling the contents of the exterior-facing display EFD to be visible. The transparent window can be coupled or configured to couple to a drape or the housing of the exterior-facing display EFD. A flexible drape material can extend from the transparent window to wrap around the exterior-facing display EFD. In another example, the housing of the exterior-facing display EFD can support a drape attachment mechanism that enables attachment of the drape to the exterior-facing display EFD. For example, the exterior-facing display EFD can define a channel that receives an elastic member (such as a flexible band) to secure the drape over the exterior-facing display EFD. In other examples, a sterile cover, sticker, or sheet sized for the exterior-facing display EFD can be installed. Any of the above implementations may be utilized in part, or in whole. Example techniques by which the exterior-facing display EFD can interface with a drape, while still providing its functionality can be like those described in U.S. Patent No. 9,713,498, entitled "Assembly For Positioning A Sterile Surgical Drape Relative To Optical Position Sensors", the entire contents of which are hereby incorporated by reference.

### 3. Other HMD Components

Additional components of the HMD 200 will now be described with reference to FIG. 2. These components can be supported by or within the support structure 202 of the HMD 200. Capabilities and functionality of these components can involve or be implemented by any one or more of the controllers 26, 42, 54, 210.

As shown in FIG. 2, the HMD 200 can include an HMD controller 210. The HMD controller 210 can include a content generator 206 that generates the computer-generated content (e.g., virtual images, graphics, text, etc.) for presentation to the user through the interior-facing display IFD. The HMD controller 210 controls the transmission of the computer-generated content to the HMD interior-facing display IFD. The HMD controller 210 may be a separate computer, located remotely from the support structure 202 of the HMD 200, and/or may be integrated into the support structure 202 of the HMD 200. The HMD controller 210 may be a laptop computer, desktop computer, microcontroller, or any suitable controller comprising memory (M), one or more processors (e.g., multi-core processors), input devices I, output devices (fixed display in addition to HMD 200), storage capability, etc.

The HMD 200 can include one or more cameras 214, as shown in FIG. 3. The cameras 214 are in communication with the HMD controller 210 and are configured to capture image or video data in the surrounding environment of the HMD 200. The image or video data can be of any object, including to detect the object to be tracked or another HMD. The image or video data can be utilized to perform numerous actions, including, but not limited to any one or more of the following: detect actions or gestures by the user of the HMD 200, detect an event or condition; modify what is presented on the HMD 200; detect presence or absence of any object, etc. By including a camera 214, the HMD 200 can perform any of the capabilities described with reference to the navigation system 20, including tracking the pose of objects, detecting surgical information or events, or the like. Cameras 214 can be built into exterior-facing display EFD of the HMD 200 and/or can be located elsewhere on the support structure 202. Multiple cameras 214, e.g., spaced from each other on the HMD 200 can provide wide-angle or stereoscopic viewing. The camera 214 can be any suitable camera, such as a 12MP or higher camera with any appropriate focal length or zooming capability.

The HMD 200 may comprise a plurality of tracking sensors 215 that are in communication with the HMD controller 210. In some cases, the tracking sensors 215 are provided to establish a global coordinate system for the HMD 200, also referred to as an HMD coordinate system. The HMD coordinate system is established by these tracking sensors 215, which may comprise camera sensors or other sensor types, in some cases combined with IR depth sensors, to layout or map the space surrounding the HMD 200, such as using structure-from-motion techniques or the like.

The HMD 200 may also comprise control input sensors 216 coupled to the HMD controller 210. In one example, the control input sensors 216 are configured to recognize gesture or eye-based commands from the user. When detecting hand-gestures, the control input sensor 216 is able to sense the user's hands, fingers, or other objects for purposes of determining the user's gesture command and controlling the HMD 200, HMD controller 210, navigation controller 26, and/or manipulator controller 54 accordingly. Gesture commands can be used for any type of input used by the system 10. The gesture commands may be detected below the HMD 200 or may be detected by the camera 214 in front of the HMD 200. The control input sensor 216 to detect gesture can include one or more cameras, infrared sensors, motion sensors, or the like. Gesture controls can include any type of hand or finger motion, including but not limited to: pinching, pointing, swiping, circling, grasping, twisting, or the like. When detecting eye-based commands, the control input sensor 216 is able to sense the user's eye position, motion, dwell time (stare), gaze and the like, for purposes of determining the user's intended command and controlling the HMD 200, HMD controller 210, navigation controller 26, and/or manipulator controller 54 accordingly. The eye-based commands may be detected using an eye-tracker that is positioned to face the user's eyes, e.g., in the interior-facing display IFD. Eye-based controls can include any type of eye-command, including but not limited to: selecting an object, moving an object, or the like. In one example, the user can select a computer-generated object displayed by the HMD 200 by staring at the object for a threshold amount of time. The HMD can also control input sensors 216 in the form of a microphone for recording verbal commands. The HMD controller 210 can process the verbal commands and control the HMD interior-facing display IFD in response. The HMD 200 could have a speaker to generate a sound or vibrate to provide an indication to the HMD user of a warning or other information of relevance.

The HMD 200 can also include one or more inertial sensors 217 coupled to the HMD controller 210. The inertial sensors 217 can be a gyroscope, accelerometer, magnetometer, MEMS device, or the like. Measurements from the inertial sensor 217 can be utilized to perform a variety of features, including but not limited to any one or more of the following: to determine a pose of the HMD 200; change an orientation of text/graphics presented on the HMD 200; communicate the measurements to the navigation system 20 to supplement tracking of the HMD 200; detect that the HMD 200 is being moved by a user; and/or detect an undesired motion of the HMD 200.

The HMD 200 may include one or more time-of-flight sensors TFS coupled to the HMD controller 210. The time-of-flight sensor TFS can be a range-finder, laser, LIDAR, or LED enabled sensor. The time-of-flight sensor TFS can also be embodied as by the camera 214 on the HMD 200. Based on the time difference between the emission of the light and its return to the time-of-flight sensor TFS after being reflected by an object, the time-of-flight sensor TFS and/or HMD controller 210 is able to measure the distance between the object and the time-of-flight sensor TFS. As will be described below, any of the one or more controllers 26, 42, 54, 210 are configured to utilize measurements from the time-of-flight sensor TFS to determine the relative spatial relationship between the HMD 200 and the navigation system 20. Additionally, or alternatively, the time-of-flight sensor TFS can be employed by the navigation system 20. A position of the HMD 200 can be determined based on the difference between the time the light is projected on the HMD 200 to the time it is detected by the time-of-flight sensor TFS. The HMD 200 can include multiple time-of-flight sensors TFS, e.g., on different sides of the HMD 200.

The HMD 200 can include one or more proximity sensors PS coupled to the HMD controller 210. The proximity sensor PS can be utilized to perform various capabilities for the HMD 200. For example, the proximity sensor PS can detect absence of environmental activity and in response place the HMD 200 or its components (e.g., EFD) in a sleep mode to conserve energy. In another example, the proximity sensor PS can detect presence of environmental activity to ensure the HMD 200 or its components (e.g., EFD) remain active. The proximity sensor PS can also function as an ambient light sensor. The proximity sensor PS can be built into the exterior-facing display EFD or can be located elsewhere on the support structure 202. In one example, the proximity sensor PS includes an infrared LED light and an IR light sensor to detect proximate activity.

The HMD 200 can include transmitter or receiver (or transceiver) (TX) that can be utilized to communicate to or from a transceiver of the navigation system 20. Such communication may be, for example, infrared communication. Communication can be performed for various purposes, such as to enable the navigation system 20 to initialize the HMD 200. Other types of communication are contemplated that may configure how the HMD 200 operates, including power settings, display settings, communication settings, authentication, sensor settings, and the like. The transceiver TX can also utilize radio frequency transmission, e.g., to communicate higher-bandwidth data, such as inertial data, and the like. Examples of IR and/or RF transceivers that can be used by the HMD 200 and navigation system 20 can be like those described in US Patent No. 10,555,781, entitled "High bandwidth and low latency hybrid communication techniques for a navigation system", the entire contents of which are hereby incorporated by reference.

Any of the described components of the HMD 200 that can sense information or process sensed information (including but not limited to, the HMD controller 210, the video camera 214, the IFD, the tracking sensors 215, the control input sensors 216, inertial sensors 217, time of flight TFS sensor, proximity sensor PS, and transceiver TX) can be understood as being part of a "sensing system" of the HMD 220.

The HMD 200 include a power source PWR. The power source PWR provides energy to power the various components of the HMD 200. The power source PWR can be a battery, such as a lithium-ion battery, alkaline battery, or the like. The power source can be rechargeable. The HMD 200 can include a charging port (e.g., USB-C connection) for enabling recharging of the power source. In some cases, the power source can be an energy harvesting power source that generates energy from motion of the HMD 200 or other sources and stores the power for use without requiring any external power source or recharging. The power source PWR can be built into the support structure 202 or removable from the support structure 202. The power source can be a battery switch, which fits into a cavity in the support structure 202. When the battery switch is rotated relative to the cavity, the battery switch is sealed to the support structure 202 and makes electrical contact to power the HMD 200. The battery switch may be disposable. The power source or battery can be like those described in: US Patent App. Pub. No. 2023/0338093, entitled "Sterilizable Surgical Device with Battery Switch", the entire contents of which are hereby incorporated by reference. In other cases, the HMD 200 may be wired to a power source PWR external to the HMD 200.

The HMD 200 may optionally also comprise one or more optical markers OM (e.g., as shown in FIG. 2). The optical markers OM can be like those described above with respect to trackers 46, 48. The optical markers OM are physical objects (not digitally presented on the exterior-facing display EFD) that may be coupled or attachable to the support structure 202 of the HMD 200. The optical markers OM may include passive markers. For example, the HMD 200 can have at least three passive tracking elements or markers (e.g., reflectors) for transmitting light signals (e.g., reflecting light emitted from the navigation system 20). In other implementations, the optical markers OM may include active tracking markers. The active markers can be, for example, light emitting diodes transmitting light, such as infrared light. Active markers may be controlled by the HMD controller 210. Active and passive arrangements are possible. The optical markers OM can be combined into a standalone tracking device that is configured to attach to the HMD 200, similar to how the trackers 46, 48 can attach to their respective objects.

As will be described below, the HMD 200 can present a trackable graphic TG on the exterior-facing display EFD to enable the navigation system 20 to track the HMD 200. The one or more optical markers OM can be used to provide communication with or supplemental tracking information to the navigation system 20. For example, such optical markers OM may be used to detect the HMD 200 in situations where the trackable graphic TG on the exterior-facing display EFD is not yet initialized or otherwise inactive. Optical markers OM can also be used to provide a supplemental form of tracking (concurrent with detection of the trackable graphic TG on the exterior-facing display EFD) for tracking redundancy and providing additional confidence in tracking. In other examples, due to the fixed relationship between the optical markers OM and the exterior-facing display EFD, the HMD 200 may operate as a registration device to facilitate registration of the HMD 200 and the localizer coordinate system. For example, the localizer 34 may detect the optical markers OM and the HMD 200 may detect the trackable graphic TG. Using the fixed relationship, the HMD 200 and localizer 34 can be registered to one another.

In some cases, a registration device may be provided with a plurality of registration markers and/or localizer markers to facilitate registering the HMD 200 to the localizer coordinate system. The localizer marker positions can be known or determined relative to the registration markers. For example, a structure can include the localization and registration markers in a fixed relationship. The HMD 200 locates the registration markers on the registration device in the HMD coordinate system via the camera 214 thereby allowing the HMD controller 210 to create a transform from the registration coordinate system to the HMD coordinate system. The localizer 34 determines the location of the localization markers in the localizer coordinate system. The HMD controller 210 then determines where the localizer coordinate system is with respect to the HMD coordinate system so that the HMD controller 210 can generate images having a relationship to objects in the localizer coordinate system or other coordinate system. The registration device or any technique for registering and/or calibrating the HMD 200 to another coordinate system can be like that described in US Patent No. 10,499,997, entitled "Systems and Methods for Surgical Navigation", the entire contents of which are hereby incorporated by reference in their entirety.

### III. SURGICAL TECHNIQUES INVOLVING EXTERIOR-FACING DISPLAY OF HMD

Described herein are various surgical implementations involving the exterior-facing display EFD of the HMD 200 being used to present text, information, graphics, etc., that can assist in surgical procedures, surgical workflow, surgical setup, tracking, and the like.

The techniques described can involve any one or more controllers that process information or signals and control the HMD 200. The one or more controllers can be remote from the HMD 200 or integrated with the HMD 200. When integrated with the HMD 200, the controller can be the HMD controller 210. Additionally, or alternatively, when remote from the HMD 200, the one or more controllers can be any suitable controller, such as the navigation controller 26, the camera controller 42, manipulator controller 54, tool controller, or a controller of a second HMD. Any of these controllers 26, 42, 54, 210 can be utilized independently or in combination for performing any of the capabilities described herein. The various controllers 26, 42, 54, 210 are configured to communicate with each other wirelessly (e.g., through Wi-Fi, Bluetooth, or any other wireless connection) or through wired connections (e.g., ethernet, etc.) and can be equipped with any appropriate hardware to enable such communications.

### 1. Presentation of Computer-Generated Trackable Graphic(s) on Exterior-Facing Display

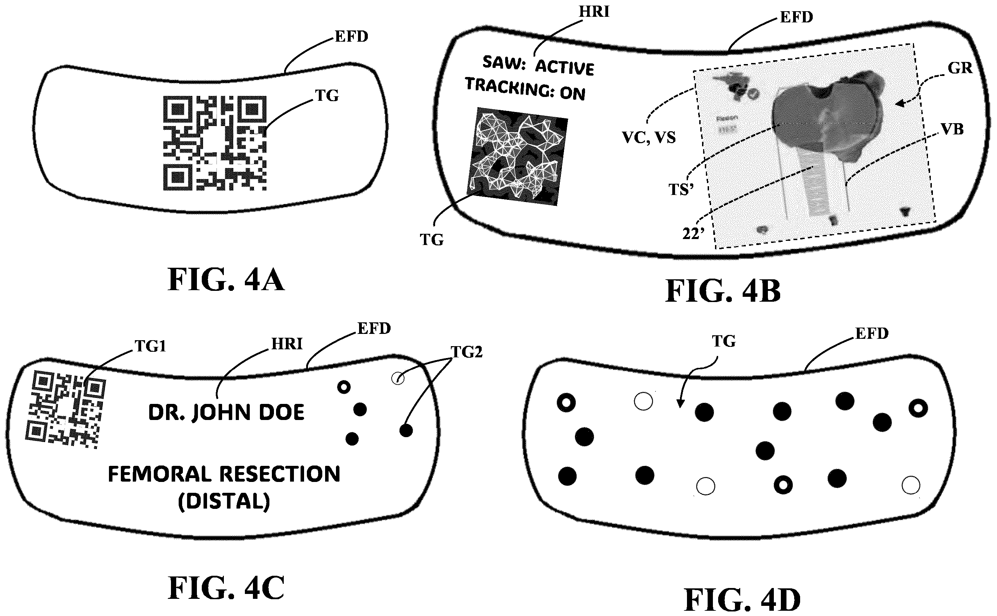

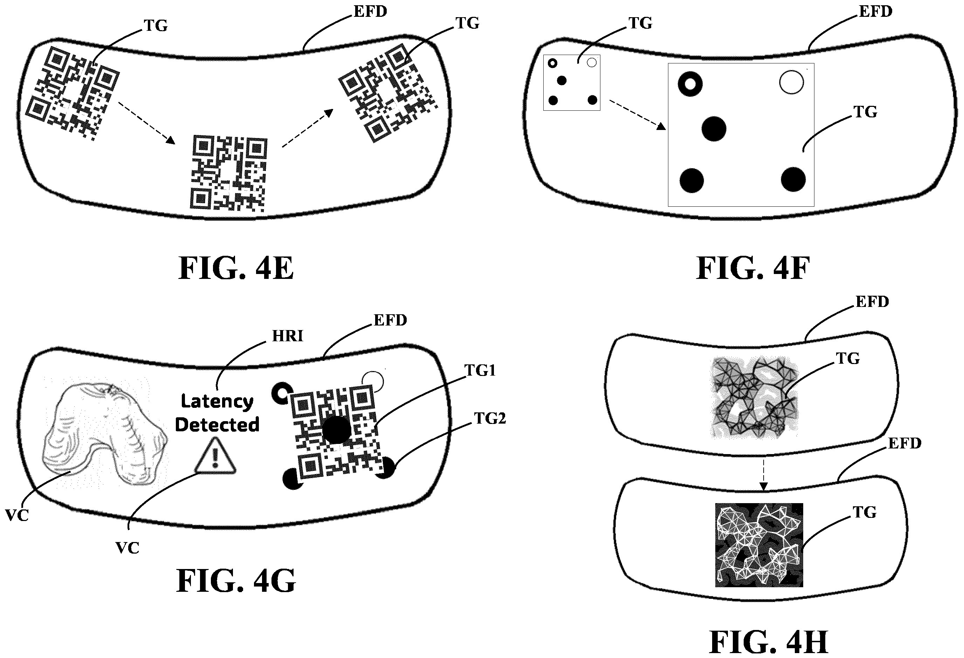

Referring now to FIGS. 4-13, described herein are techniques whereby the HMD 200 presents a computer-generated trackable graphic TG on the exterior-facing display EFD. The display of the trackable graphic TG can be so that a remote camera source can detect the trackable graphic TG. The remote camera source can be any one or more tracking systems that can detect graphics presented by the exterior-facing display EFD. Examples of such tracking systems include but are not limited to: optical tracking systems, such as the localizer 34 (or camera) of the navigation system 20, a camera coupled to a surgical tool 22, (such as an endoscope, a laparoscopic, arthroscope, hand-held robotic tool, etc.), a camera coupled to the manipulator 14, or a camera of a second HMD (which will be described below). For simplicity in description, any one or more of these tracking systems will be referred to as the tracking system TRKSYS.

By presenting the trackable graphic TG, the tracking system TRKSYS detecting the trackable graphic TG can facilitate tracking of a pose of the HMD 200. The trackable graphic TG can be presented in a manner configured to enable the tracking system TRKSYS to determine the pose of the HMD 200 in at least five-degrees of freedom, and up to six degrees of freedom. One trackable graphic TG may be simultaneously detectable by multiple tracking systems TRKSYS. Multiple trackable graphics TG may be simultaneously detectable by one or more tracking systems TRKSYS. In one implementation, the HMD controller 210 implements control and instructions to present the trackable graphic TG on the exterior-facing display EFD. However, as described, control and presentation of the trackable graphic TG on the exterior-facing display EFD can additionally or alternatively be implemented by any one or more other controller(s) 26, 42, 54, 210.

As shown in at least the examples of FIGS. 4A-4H, the trackable graphic TG can be presented on or more exterior-facing displays EFD of the HMD 200 according to numerous manners and forms. For example, one exterior-facing display EFD can present one or multiple trackable graphics TG at the same time or separate times. Multiple exterior-facing displays EFD can present the same trackable graphic TG. Multiple exterior-facing displays EFD can present different trackable graphics TG. The trackable graphic TG can occupy an entirety or a portion of any exterior-facing display EFD area. The exterior-facing display EFD can present any appropriate background or surround in conjunction with the trackable graphic TG, e.g., to make the trackable graphic TG more visible. For example, the background can be white/black or can be updated based on detected environmental or ambient conditions. One trackable graphic TG can span, extend across, or move across multiple exterior-facing displays EFD. When the exterior-facing display EFD is curved, the trackable graphic TG can be presented in a curved manner on the exterior-facing display EFD and can move about the curved exterior-facing display EFD. A configuration or geometry of the trackable graphic TG may be configured to change, e.g., during use of the HMD 200. One exterior-facing display EFD may toggle on/off any trackable graphic TG. Any one or more trackable graphics TG may be swapped for presentation between one exterior-facing display EFD and another exterior-facing display EFD. The trackable graphic TG may be static or may change pose on one or more exterior-facing displays EFD. The trackable graphic TG may change size or shape on one or more exterior-facing displays EFD. The trackable graphic TG may move about one or more exterior-facing displays EFD. The trackable graphic TG may change form on one or more exterior-facing displays EFD.

The trackable graphic TG can be optimized or customized for the respective tracking system TRKSYS detecting the HMD 200. Such optimization can include any of the described implementations explaining when, where and how to configure and/or display the trackable graphic TG on the exterior-facing display EFD. In one example, the intensity or frequency of the light emitted by the exterior-facing display EFD may change. Light intensity or frequency may be changed, for example, to be optimized for the tracking system TRKSYS the HMD 200 is working with. Given the various lighting systems in the operating room, the intensity or frequency and display graphics settings could be optimized for every procedure, for every location of the device, or based on changing lighting conditions in the surrounding area at different times during a procedure. In other examples, the HMD 200 can present multiple trackable graphic TG that can be detected by different tracking systems TRKSYS. For example, one portion of the exterior-facing display EFD can present a first trackable graphic TG for a first tracking system TRKSYS and a second portion of the exterior-facing display EFD can present a second trackable graphic TG for a second tracking system TRKSYS. The trackable graphics TG can be presented side-by-side, on different sides, or overlapping one another. In another example, trackable graphics TG can be displayed in an alternating or strobing manner, whereby the exterior-facing display EFD alternates presentation of different trackable graphics TG on the same region of the exterior-facing display EFD. Any of these manners of controlling the trackable graphic TG can be utilized individually or in combination.

As will be described below, the user may utilize a graphical user interface GUI implemented by the HMD 200 to control settings to specify when, how, and where the trackable graphic(s) TG, or any other content or information is/are presented on the exterior-facing display(s) EFD.

FIGS. 4A-4H illustrate numerous examples of the trackable graphic TG. For example, as shown in FIG. 4A, the trackable graphic TG comprises a QR code. The QR code can be any type of QR code, such as a Model 1 QR code, Mirco QR code, iQR code, Secure Quick Response (SQR) code, Frame QR code, or a High Capacity Colored 2-Dimensional (HCC2D) code, or the like. In some cases, the QR code can be a dynamic QR code encoded with supplemental information or instructions for the tracking system TRKSYS. The one or more controllers 26, 42, 54, 210 can repeatedly change the QR code or dynamic QR code during tracking. For example, the size, shape, configuration, intensity, density, and/or color of the codes can be changed. In one implementation, the QR code is implemented as a particle cloud of points that dynamically change shape and/or color. FIG. 4A shows one QR code centered on the exterior-facing display EFD of the HMD 200. FIG. 4C one QR codes located on a right-side of the exterior-facing display EFD and having a reduced size compared to the QR code of FIG. 4A. As described, the exterior-facing display EFD can display multiple QR codes that can be the same or different from one another. Of course, one or more QR codes can be presented in various manners depending on the configuration of the HMD 200, exterior-facing display(s) EFD, and/or conditions affecting presentation.

In another example, FIGS. 4C and 4D illustrate the trackable graphic TG as digital fiducials or markers. These digital fiducials or markers are digitally presented on the exterior-facing display EFD, and hence, are different from the physical optical markers OM, shown coupled to the HMD 200 in FIG. 2, for example. The digital fiducials may, but need not, be infrared detectable fiducials. The digital fiducials can be presented in any number, style, and arrangement. The digital fiducials can be various sizes, colors, and shapes. The one or more controllers 26, 42, 54, 210 can repeatedly change the features of the digital fiducials during tracking. For example, the size, shape, configuration, intensity, density, and/or color of the digital fiducials can be changed. FIG. 4C shows digital fiducials on the right-hand side of the exterior-facing display EFD. In FIG. 4D, the digital fiducials extend across a substantial entirety of the exterior-facing display EFD. At least three digital fiducials are presented, which can help the tracking system TRACKSYS detect the pose of the HMD 200 in at least five degrees of freedom. The digital fiducials are shown as circles or dots, with different filling or outlining. A curved or multi-faced exterior-facing display EFD with digital fiducials may further improve tracking accuracy. For example, multiple digital fiducials can be presented on a spherical-shaped exterior-facing display EFD. The digital fiducials may be static or may move about the spherical exterior-facing display EFD. If a cube shaped exterior-facing display EFD is used, the multiple exterior-facing displays EFD may each show the same digital fiducial arrangement or a different digital fiducial arrangement. In another example, each exterior-facing display EFD may show one or more digital fiducials, which when viewed collectively among the exterior-facing displays EFD, form the arrangement of digital fiducials. Of course, digital fiducials can be presented in various other manners depending on the configuration of the HMD 200, exterior-facing display(s) EFD, and/or conditions affecting presentation.

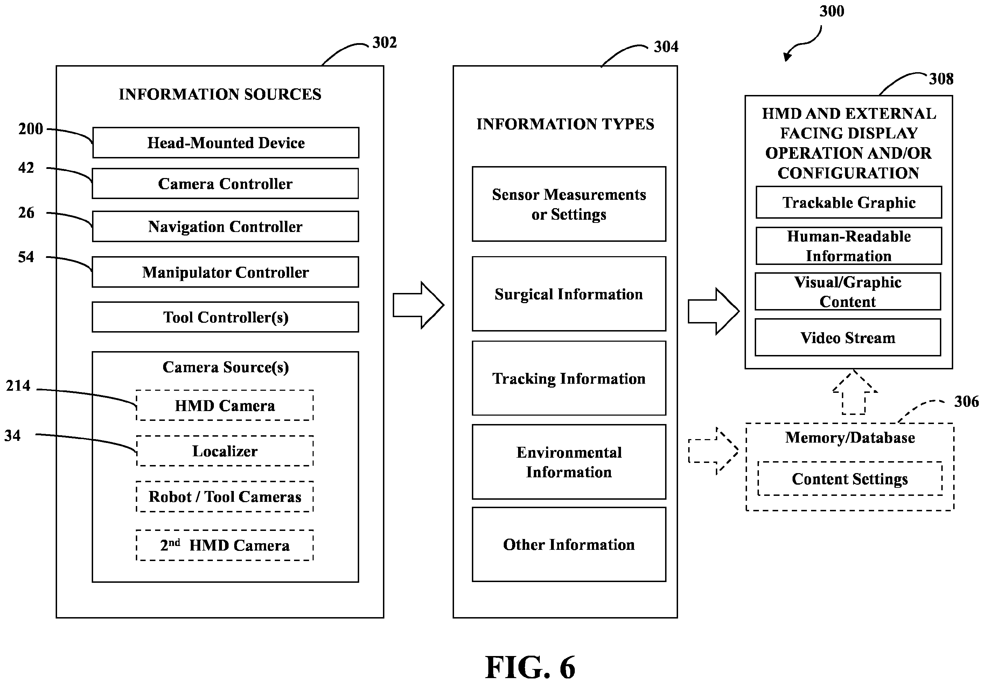

In one implementation, the digital fiducials can be arranged or presented on the exterior-facing display EFD to mimic predetermined geometries of physical optical markers OM for other tracking devices. For example, as will be described with respect to FIG. 6, the one or more controllers 26, 42, 54, 210 can obtain information and look up, in a memory or database, predetermined geometries of physical optical markers OM for other tracking devices. From this obtained information, the one or more controllers 26, 42, 54, 210 can transform the predetermined geometry of physical optical markers OM into the geometry and arrangement of the digital fiducials on the exterior-facing display EFD.

FIGS. 4B and 4H illustrate other examples wherein the trackable graphics TG are implemented as static or dynamically changing point clouds. Other examples of trackable graphics TG are contemplated, including but not limited to any bar codes, icons, graphics, text, images, video, or any predefined or irregular arrangement of points, lines, planes, or geometry that can be detected by the tracking system TRKSYS to track a pose of the HMD 200.

The trackable graphic TG is configured to move about the exterior-facing display EFD. For example, as shown in FIG. 4E, the trackable graphic TG is moved from the upper left-hand corner of the exterior-facing display EFD to the bottom of the exterior-facing display EFD and ultimately to the upper-right hand corner of the exterior-facing display EFD. Movement of the trackable graphic TG is denoted by a dashed arrow. Notably, in this example, the orientation of the trackable graphic TG changes between the positions. The trackable graphic TG can be rotated, turn, flipped, inverted, or moved in any way to modify its pose. In FIG. 4E, the trackable graphic TG remains the same size during movement. However, in other examples, as shown in FIG. 4F, for instance, the trackable graphic moves and changes size. In FIG. 4F, the trackable graphic TG is configured to change size. The trackable graphic TG is presented in the upper left corner of the exterior-facing display EFD and then is simultaneously enlarged and moved to the center of the exterior-facing display EFD. Such movement, pose changes, and size changes of the trackable graphic TG can be performed one or more discrete times, or continuously during use of the trackable graphic TG. Also, movement, pose and size changes can occur for multiple trackable graphics TG and with respect to one or more exterior-facing displays EFD.

FIG. 4G illustrates an example whereby the multiple trackable graphics TG1, TG2 are presented in the same region of the exterior-facing display EFD. The trackable graphics TG1, TG2, can be the same type or different types. Here, a first trackable graphic TG1 is a QR code and a second trackable graphic TG2 is an arrangement of digital fiducials. In one example, trackable graphics TG1, TG2 can be displayed in an alternating or strobing manner, whereby the exterior-facing display EFD alternates presentation of trackable graphics TG1, TG2 on the same region of the exterior-facing display EFD. This alternating or strobing can be at any frequency, such as at 60 Hz or greater. In another example, the trackable graphics TG1, TG2 can be combined in one trackable graphic that combines both types of graphics (e.g., QR code and digital fiducials overlapped). In such situations, the combined trackable graphic TG1, TG2 can be continuously presented (e.g., without alternating presentation). These techniques can be utilized with any number of trackable graphics TG and with respect to one or more exterior-facing displays EFD.

FIG. 4H illustrates an example whereby display properties of the trackable graphic TG are changed. Any display settings of the trackable graphic TG can be modified. Here, for example, the trackable graphic TG is a point cloud type. In FIG. 4H, the same exterior-facing display EFD is shown at two different times. Between the first time and the second time, the color, brightness, and contrast of the trackable graphic TG is changed on the exterior-facing display EFD. Of course, as described herein, other display properties of the trackable graphic TG can be modified, such as but not limited to: intensity, density, illumination, display rate (frequency), hue, strobing, resolution, display orientation, HDR setting, scaling, graphic position, focus, display format, or the like.

These examples of the trackable graphics TG are provided solely for illustration. The trackable graphics TG will change depending on the specific configuration and application of use. Additional configurations, uses, and capabilities of the exterior-facing display EFD and the trackable graphic TG will be further understood and described in sections below. Also, the sources and inputs which related to generation of the trackable graphics TG will be described in greater detail below.

### 2. Presentation of Other Content on Exterior-Facing Display of HMD

The HMD 200 can additionally or alternatively present other information or content on the exterior-facing display EFD. This other content can be supplemental or non-trackable information or content, which can be presented with or without presenting the trackable graphic TG. The various controllers, sensors, configurations, and inputs described above can be used to detect, generate, configure, and present the following other content on the exterior-facing display EFD.

Advantageously, the described techniques can provide surgically relevant information, text, graphics, or video directly on exterior-facing display EFD of the HMD 200 so that others can readily observe this content without needing to look away at external displays to find such information. The exterior-facing display EFD will be readily visible to others when the wear is using the HMD 200 at the surgical site. In turn, this technique can prevent impairing the view of surgical staff and can minimize disruption or distraction to the surgical staff.

### i. Human-Readable Content

For example, human-readable information HRI can be presented on the exterior-facing display EFD. The human-readable information HRI includes text, messages, notifications, alerts, warning, images, or graphics that can be naturally read by the user and can be based on any information source or information type, such as but not limited to any one or more of: surgical information, HMD user identity, patient information, operating instructions, information about a surgical object, an identity of a surgical object, a status of the HMD 200, a status of the tracking system TRKSYS, information about a surgical procedure or step of the surgical procedure, surgical workflow information, surgical plan information, and a warning or alert related to the surgical procedure or tool, and the like.

The human-readable information HRI can be presented on or more exterior-facing displays EFD of the HMD 200 according to numerous manners and forms and can be like those described above with respect to how the trackable graphic TG can be presented. For example, the human-readable information HRI can be generated or modified by shape, size, position, orientation, font, display parameters, etc. One exterior-facing display EFD can present one or multiple human-readable information HRI at the same time or separate times. Multiple exterior-facing displays EFD can present the same human-readable information HRI. Multiple exterior-facing displays EFD can present different human-readable information HRI. The human-readable information HRI can occupy an entirety or a portion of any exterior-facing display EFD area. The exterior-facing display EFD can present any appropriate background or surround in conjunction with the human-readable information HRI, e.g., to make the human-readable information HRI more visible. For example, the background can be white/black or can be updated based on detected environmental or ambient conditions. One human-readable information HRI can span, extend across, or move across multiple exterior-facing displays EFD. When the exterior-facing display EFD is curved, the human-readable information HRI can be presented in a curved manner on the exterior-facing display EFD and can move about the curved exterior-facing display EFD. A configuration or geometry of the human-readable information HRI may be configured to change, e.g., during use of the HMD 200. One exterior-facing display EFD may toggle on/off any human-readable information HRI. Any one or more human-readable information HRI may be swapped for presentation between one exterior-facing display EFD and another exterior-facing display EFD. The human-readable information HRI may be static or may change pose on one or more exterior-facing displays EFD. The human-readable information HRI may change size or shape on one or more exterior-facing displays EFD. The human-readable information HRI may move about one or more exterior-facing displays EFD. The human-readable information HRI may change form on one or more exterior-facing displays EFD. Human-readable information HRI may be presented concurrently with the trackable graphic TG or at a different time from presentation of the trackable graphic TG. Human-readable information HRI and the trackable graphic TG can be presented on one or multiple exterior-facing display EFD concurrently. One exterior-facing display EFD can present human-readable information HRI and a second exterior-facing display EFD can present the trackable graphics TG, at the same or separate times. Multiple exterior-facing displays EFD can present the same or distinct types of human-readable information HRI. Any of these manners of controlling the human-readable information HRI can be utilized individually or in combination. The user may utilize the graphical user interface GUI implemented by the HMD 200 to control settings to specify what, when, how, and where the human-readable information HRI is/are presented on the exterior-facing display(s) EFD.

In FIG. 4B, for example, the human-readable information HRI is a status of a tool 22 utilized by the user or the HMD 200 or another user in the operating room. The human-readable information HRI displays "Saw: Active" to signify that the tool is a saw and the saw is actively being used. This status may mean the tool 22 is being actuated and/or tracked by the tracking system TRKSYS. Another human-readable information HRI displayed in FIG. 4B is a tracking status "Tracking: On" to signify that the HMD 200 is actively being tracked by the tracking system TRKSYS using the trackable graphic TG that is presented on the exterior-facing display EFD. In FIG. 4C, the human-readable information HRI includes the name of the physician wearing the HMD 200, i.e., "Dr. John Doe". Displaying such identifying information may be beneficial to others in the operating room, particularly when staff are wearing surgical masks or helmets that may obstruct the identity of the staff. Also displayed on the exterior facing display EFD is human-readable information HRI that identifies a particular step of the procedure, e.g., "Femoral Resection (Distal)." Display surgical steps can help others in the operating room confirm the correct surgical step and/or prepare the necessary tools or surgical setup for this step. In FIG. 4G, the human-readable information HRI includes a warning message that latency is detected. The latency can be between the HMD 200 and the tracking system TRKSYS, for instance. Other warnings or alerts can be presented in human-readable form on the exterior-facing display EFD. Human-readable information HRI can also be embedded or presented within visual objects, pictures, graphics, or video on the exterior facing display EFD.

These examples are provided solely for illustration. The wording and nature of the human-readable information HRI will change depending on the specific configuration and application of use. The sources and inputs which related to generation of the human-readable information HRI will be described in greater detail below.

### ii. Visual/Graphic Content

Also, visual/graphic content VC can be presented on the exterior-facing display EFD. Visual/graphic content VC can include pre-captured images, live-stream video feeds or real-time captured images, pre-recorded video or images, pictures, computer-generated 2D/3D graphics, and the like. The visual/graphic content VC can be based on any information source or information type, such as but not limited to any one or more of: surgical plan information, information of or about any surgical object, imagery of an anatomy, imagery of an implant, medical imaging data, video data from a camera, icons, a video stream provided from a software application of a device in the operating room (e.g., the clinical application CA), a warning or alert information.

This visual/graphic content VC can be presented on the exterior-facing display EFD according to numerous manners and forms. Visual/graphic content VC may be presented concurrently with the trackable graphic TG or human-readable information HRI or at a different time from presentation of the TG or HRI. Visual/graphic content VC and the TG or HRI can be presented on one or multiple exterior-facing display EFD concurrently. One exterior-facing display EFD can present visual/graphic content VC and a second exterior-facing display EFD can present the TG or HRI, at the same or separate times. Multiple exterior-facing displays EFD can present the same or distinct types of visual/graphic content VC. The visual/graphic content VC can occupy an entirety or a portion of any exterior-facing display EFD area. Visual/graphic content VC can span, extend across, or move across multiple exterior-facing displays EFD. When the exterior-facing display EFD is curved, the visual/graphic content VC can be presented in a curved manner on the exterior-facing display EFD and can move about the curved exterior-facing display EFD. A configuration or geometry of the visual/graphic content VC may be configured to change, e.g., during use of the HMD 200. One exterior-facing display EFD may toggle on/off any visual/graphic content VC. Any one or more types of visual/graphic content VC may be swapped for presentation between one exterior-facing display EFD and another exterior-facing display EFD. The visual/graphic content VC may be static or may change pose on one or more exterior-facing displays EFD. The visual/graphic content VC may change size or shape on one or more exterior-facing displays EFD. The visual/graphic content VC may move about one or more exterior-facing displays EFD. The visual/graphic content VC may change form on one or more exterior-facing displays EFD. Any of these manners of controlling the visual/graphic content VC can be utilized individually or in combination. The user may utilize the graphical user interface GUI implemented by the HMD 200 to control settings to specify what, when, how, and where the visual/graphic content VC is/are presented on the exterior-facing display(s) EFD.

In FIG. 4B, the exterior-facing display EFD presents visual/graphic content VC in the form of a live-video stream VS that is obtained from a software application run by equipment in the operating room, such as the clinical application CA run by the navigation system 20. This video stream VS will be described in greater detail below. In FIG. 4G, the exterior-facing display EFD presents visual/graphic content VC in the form of a virtual bone model for the patient and/or target site TS. This may be beneficial to provide confirmation to others regarding the target site TS. Also presented is visual/graphic content VC in the form of an alert icon, which relates to a latency issue. Again, any type of alert or warning icons can be displayed for any appropriate warning or notification.

These examples of the visual/graphic content VC are provided solely for illustration. The visual/graphic content VC will change depending on the specific configuration and application of use. Additional configurations, uses, and capabilities of the exterior-facing display EFD and the visual/graphic content VC will be further understood and described in sections below. Also, the sources and inputs which related to generation of the visual/graphic content VC will be described in greater detail below.

### iii. Video Stream

As shown in FIG. 4B, the exterior-facing display EFD can present a video stream VS or portion thereof that is directly obtained from a piece of equipment that hosts and runs a software application. Surgical information can be identified and/or extracted from any host system/device (e.g., in the operating room) that is configured to display the software application, such as the clinical application CA presented by the navigation system 20. The video stream VS may be wirelessly transmitted or communicated to the HMD 200 using a wired connection.

The host system/device 20 and respective software application can take various forms. For example, the host system/device 20 and software application can include any of: an endoscopic system that operates a software application for the endoscopic system; an imaging system (e.g., CT scanner) that operates a software application for the imaging system; a (CORE) console that operates a software application for operation of powered instruments; a surgical robot that operates a software application for controlling the surgical robot, a hand-held tool that operates a software application for controlling the hand-held tool, a surgical visualization system (e.g., arthroscope, ultrasound, laparoscope) that operates a software application for controlling the surgical visualization system, a surgical waste management system that operates a software application for controlling the surgical waste management system, a fluid management system that operates a software application for controlling the fluid management system, a sponge management system that operates a software application for controlling the sponge management system, a patient support apparatus that operates a software application for controlling the patient support apparatus, and the like.

In one implementation, any of the described controllers 26, 42, 54, 210 can utilize a stream analyzer to process the video stream VS and recognize the surgical information therein by automatically identifying text presented by the software application. The stream analyzer can utilize any text recognition algorithm to perform this function, such as optical character recognition OCR, visual text recognition, scene text recognition, natural language processing NLP, any combination thereof, and the like. Additionally, or alternatively, any of the described controllers can utilize the stream analyzer to recognize the surgical information by automatically identifying imagery or graphics presented by the software application. The stream analyzer can utilize any image recognition algorithm to perform this function, such as segmentation, bounding boxes, pattern recognition, shape modeling, machine learning models, deep learning, neural networks, convolutional neural networks, any combination thereof, and the like. Additionally, or alternatively, the one or more controllers 26, 42, 54, 210 can utilize the stream analyzer to recognize the surgical information by automatically identifying user inputs provided on the software/clinical application. Such inputs may include mouse movements or behavior, cursor selections, inputted text (e.g., using a keyboard), screen selections, icon selections, movement, or manipulation of graphical objects, such as scroll bars, up/down arrows, bone models, implants, and the like. Video streams can be analyzed according to any technique described in U.S. Provisional Patent App. No. 63/551,719, filed February 9, 2024, and entitled "Extended Reality Systems and Methods for Surgical Applications", the entire contents of which are hereby incorporated by reference.

The surgical information identified or extracted from the video stream VS by the one or more controllers 26, 42, 54, 210 may include any information that may be relevant to the surgeon, patient, or surgical procedure. The surgical information may, but need not, be related to the process of actually performing surgery. The surgical information can be pre-operative surgical information. Alternatively, surgical information can include post-operative information, such as reports, etc. Examples of surgical information include but are not limited to: patient information, medical images (e.g., CT scan or volume, X-rays, etc.), surgical guidance information (e.g., tool interaction with target site), surgical planning information, an anatomical model, an implant model, a cut plan, a resection plan or volume, a virtual boundary VB or cutting boundary, surgical tool information, operating room or tool setup information, surgical step information, clinical application information, surgical alerts, notifications or warnings, and the like. The surgical information can be a step of the surgical procedure. The step of the surgical procedure can include but are not limited to: a pre-operative planning step, an operating room setup step, an anatomical registration step, an intra-operative planning step, an anatomical preparation step, or a post-operative evaluation step. The surgical information detected can include initialization, progression, or completion of any surgical step. The surgical information detected can include a time component or duration defining presence or absence of any of the described surgical information.

The information identified and/or extracted from the software/clinical application of the host system/device depends on what the software/clinical application is configured to present. For example, the clinical application CA can have a plurality of different screens related to the surgical procedure, such as, but not limited to: "pre-op check," "bone registration," "intra-op planning," "bone preparation" and "case completion." Of course, the exact wording of the screen may vary depending on the clinical application CA use case. Each screen can have a screen identifier. Sometimes, the screen identifier can be a title of the screen, such as the "bone preparation" text. In one example, the one or more controllers 26, 42, 54, 210 can utilize the stream analyzer to automatically identify the screen identifier of the active one of the screens of the clinical application CA. This can be performed by identifying the text of the title and/or by identifying any other graphic or text that can identify the contents of the screen. The information that identifies the screen can be located anywhere in the screen. Alternatively, the stream analyzer can specifically monitor changes in information provided only within a specified region on the screen. In another example, a detection region could be defined around a location on the screen where an icon or a visual indicator is/would be displayed. Additionally, or alternatively, the one or more controllers 26, 42, 54, 210 can use the stream analyzer to identify or extract any information from the clinical application CA, regardless of identifying the specific screen on which such information is presented. For example, the stream analyzer can detect certain text/graphics that may be unique to the particular screen. The stream analyzer may detect the word "tibia" to understand the context or contents of the screen, e.g., that this screen involves bone preparation for the tibia (as compared to the femur, for example).

In some implementations, certain regions of the screen can be monitored from the video stream VS to detect surgical information that subsequently triggers monitoring or detection of another region of the screen. For example, the detection of specific text on the screen can trigger clipping or reproduction of a graphical part of the screen for presentation on the exterior-facing display EFD of the HMD 200.

Referring back to FIG. 4B, the HMD 200 could implement presentation of the video stream VS by reproducing a guidance region GR on the exterior-facing display EFD. The guidance region GR can display one or more surgical objects tracked by the tracking system TRKSYS. Bounding boxes or detection regions can be used to define boundaries of the region to clip or reproduce from the video stream VS for presentation on the exterior-facing display EFD. The exterior-facing display EFD presents a portion of video stream VS that is directly obtained from the clinical application CA run by the navigation system 20, according to the techniques described above. For example, the HMD 200 may receive a real-time video stream VS of the clinical application CA that shows the guidance region GR presenting interaction between a virtual representation of the tool 22' and a virtual representation of the target site TS' and a virtual boundary VB for guiding resection of the target site TS'. The relative spatial relationship between these virtual representations of the tool 22' and target site TS' track, in real-time, the relative spatial relationship between the physical tool 22 and target site TS obtained from the localizer 34. The HMD 200 may display this guidance region GR in response to detection of certain events, e.g., in response to the one or more controllers: detecting the appropriate step of the procedure; detecting operation of the tool 22; and/or detecting text/graphics from the clinical application CA using the stream analyzer.

Hence, the HMD 200 is configured to intelligently present content on the exterior-facing display at appropriate or relevant times. Advantageously, as understood from this example, by having the video stream VS presented directly on the exterior-facing display EFD, other surgeons or staff can easily visualize relevant information directly on the HMD 200 located directly next to the target site TS or tool 22 without needing to look away, e.g., to an external monitor. This benefit of the HMD 200 helps reduce distractions during surgery and can help save time during surgery by dynamically providing any relevant information to the surgeon, without the surgeon asking for such information or looking for such information.

Additionally, this video stream VS technique provides an intuitive and selective configuration dictating how information or how much information is displayed on the exterior-facing display EFD of the HMD 200. The described techniques can display the information that the surgical staff needs when they need such information and where they need such information to be displayed. In turn, the described techniques avoid displaying an overwhelming amount of information and avoid displaying information in undesirable location. Also, by monitoring certain regions of the software application and/or clipping or reproducing certain portions of the software application, the described techniques advantageously improve performance of the system, speed of processing information, and recognition accuracy. Namely, by monitoring certain regions for information, the one or more controllers need not waste resources on monitoring the entirety of the video stream VS contents and can utilize its processing power for other purposes. Additionally, by clipping or reproducing only certain regions of the software application for presentation, the system can process and present such content on the exterior-facing display EFD of the HMD 200 much faster than if the entire software screen were to be reproduced. Hence, these techniques further reduce the latency in presentation of video data to the exterior-facing display EFD of the HMD 200.

### 3. Inputs Influencing Presentation of Surgical Content on Exterior-Facing Display

FIG. 6 shows an example method 300 illustrating numerous ways to operate and/or configure the HMD 200 or exterior-facing display EFD to present content thereon. For example, the described sources/inputs may affect what, when, where, and how content is presented by the exterior-facing display EFD. The following description applies to all described content that can presented on the exterior-facing display EFD, including the trackable graphic TG, human-readable information HRI, visual/graphic content VC, video stream VS, or any other information (collectively "content"). These described techniques may be optional and the HMD 200 may be pre-configured without requiring additional input. The techniques described in FIG. 6 may be performed pre-operatively or intra-operatively. Any of these techniques may cause automatic configuration or operational changes to the HMD 200 or EFD, e.g., without user input. In other scenarios, the user may provide input configuration or operational changes to the HMD 200 or EFD.

At 302, information can be provided from one of a plurality of information sources, which can provide information dictating or influencing display of the content on exterior-facing display EFD. The sources of information include any of the described components of the system 10 and other sources, such as but not limited to: the HMD 200 itself, the camera controller 42, the navigation controller 26, the manipulator controller 54, the tool controller, or any camera source, such as the camera 214 of the HMD 200, a camera 214 of the HMD 200, a camera attached to any surgical tool 22 or device (e.g., endoscope, laparoscope, arthroscope), or a camera attached to the manipulator 14. Additional sources may include other HMDs located in the operating room, which can comprise any of the described features and capabilities of the HMD 200 described herein, including a camera and exterior-facing display EFD.

At 304, information is provided from any one or more of the information sources. The information can be of diverse types depending on the source. The information can include signals, data, commands, measurements, controller determinations, settings, and the like. For example, the information from any one or more of the described information sources can include but are not limited to: sensor measurements or settings; surgical information; tracking information; environmental information; or any other type of information. Any of these information types can be utilized individually or in combination, and the information can be obtained from any one or more of information source and at any given time.

### i. Information from HMD

The HMD 200 can configure and provide input for presentation of the content on exterior-facing display EFD in numerous ways. For example, the HMD 200 can utilize measurements or data from any of its described hardware, e.g., HMD controller 210, video camera 214, interior-facing display IFD, exterior-facing display EFD, tracking sensors 215, control inputs 216, inertial sensors 217, time-of-flight sensor TFS, transceiver TX, proximity sensor PS, or power source PWR. Similarly, any measurements or data from the HMD 200 can be obtained, such as from the or the like.

The camera(s) 214 of the HMD 200 may influence when or how content is displayed on the exterior-facing display EFD. For example, the camera 214 of the HMD 200 can acquire image or video data. Such image or video can be of virtually anything in the operating room, including but not limited to: any surgical object, the target site, the tracking system TRKSYS, optical markers OM attached to any object, surgical actions (e.g., tool properties, operation, or movements, anatomy properties or movements, interaction between objects, and the like). In one case, the images or video data can be directly presented on the exterior-facing display EFD. In another instance, the acquired images or video may be processed by any of the described controllers for deriving information, surgical information or surgical context from such images or video. The derived information or context can also be presented on the exterior-facing display EFD.

The camera 214 can detect presence, absence, or movement of any surgical object or person. In response a confirmation or warning message may be generated on the exterior-facing display EFD. Presence, absence, or movement of the tracking system TRKSYS can be similarly detected for presenting relevant content on the exterior-facing display EFD. For example, the trackable graphic TG may be presented when the tracking system TRKSYS is present, and the trackable graphic TG may not be presented when the tracking system TRKSYS is absent. In another example, the camera 214 may detect the type of tracking system TRKSYS, which can trigger presentation of a trackable graphic TG specifically adapted for the type of tracking system TRKSYS. The camera 214 can detect line-of-sight obstructions between the HMD 200 and the tracking system TRKSYS. This detected obstruction may cause movement of the trackable graphic TG to a new location one or more exterior-facing displays EFD or may cause display of a warning or alert.

In some cases, the HMD 200 camera 214 can detect a second HMD 200. For example, the second HMD 200 may present a trackable graphic TG that is detected by the camera 214 of the HMD 200. The relative spatial relationship between the two tracker devices TD can be determined. This technique can be performed in a "daisychain" fashion among several tracker devices TD to determine more complex transforms or relationships between objects in the operating room. The camera 214 can also detect a face of a user to authenticate use of the HMD 200. Authentication may be implemented to ensure the HMD 200 is used by the specified surgeon or for a specific procedure or patient. Facial recognition software may be implemented by the HMD controller 210 to perform this task. This task can be performed before or during surgery.

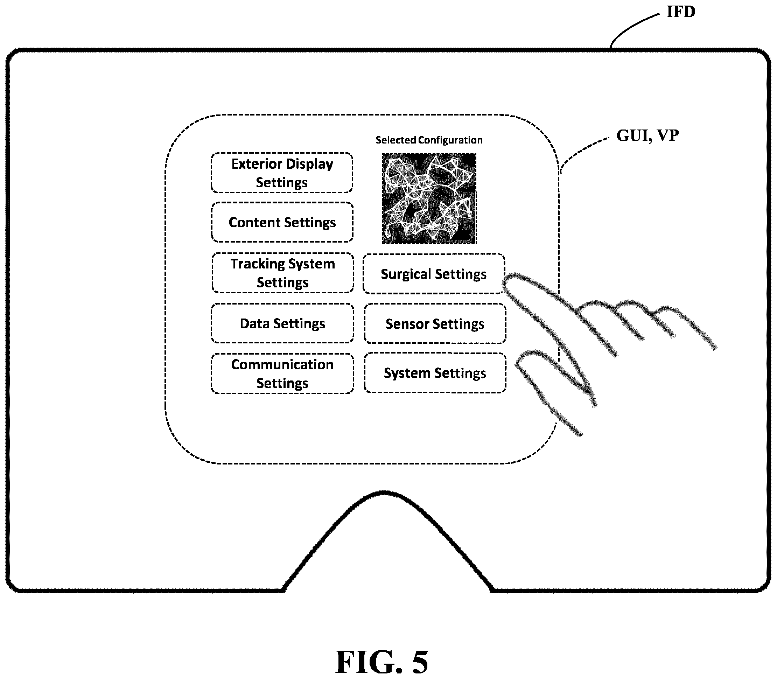

As shown in FIG. 5, the HMD 200 wearer or another person may utilize a graphical user interface GUI implemented by the HMD 200 to provide input, for example, to modify settings or operation of the HMD 200. For instance, the GUI enables the user to control settings to specify what, when, how, and/or where content is presented on the exterior-facing display(s) EFD. The GUI may be presented to the wearer of the HMD 200 using the interior-facing display IFD, as shown in FIG. 5. Additionally, or alternatively, the GUI may be presented using the exterior-facing display EFD. The GUI can be manipulated on the exterior-facing display EFD before use of the HMD 200 (e.g., by the wearer of the HMD) or during use of the HMD 200 (e.g., by an assistant or other individual). On the exterior-facing display EFD, the GUI may be interfaced with by a touch-screen display or any other type of user input. On the interior-facing display IFD, the GUI may be implemented as a virtual object that is combined with real-world views and the HMD 200 wearer can use control inputs 216 such as gaze or gesture to manipulate the GUI.

The GUI can provide various settings, including but not limited to exterior display settings (EFD), content settings, tracking system settings, data settings, communication settings, surgical settings, sensor settings, system settings, and the like. Display settings can configure any parameters of the exterior-facing display EFD, such as intensity, frequency, density, illumination, brightness, hue, color, strobing, resolution, display orientation, HDR setting, scaling, contrast, graphic position, focus, display format, idle timeout, or the like. Content settings can be used to configured any of the described implementations of the content presentable on the EFD. For example, the size, shape, type, or location of the content can be specifically set. When the HMD 200 has multiple EFDs, the settings can also specify what exterior-facing display EFD will present the content. Tracking system settings may define an identity or type of the tracking system TRKSYS being utilized with the HMD 200, the type of connection or spatial relationship between the HMD 200 and the tracking system TRKSYS, the tracking configuration or modality, and the like. Data settings can define what data is received by the HMD 200 as well as how/when such data. This data can be from the various information sources described in FIG. 6. Data settings can also include selections regarding acquisition of protected health information. Communication settings can define parameters of communication between the HMD 200 and any other system. For example, communication settings could define whether or not the HMD 200 can communicate information to a remote server. Remote server communication can be for providing software updates to the HMD 200 and/or for transmitting acquired information from the HMD 200. Other communication settings, such as transceiver TX settings, IR/RF communication settings, can be specified. Surgical settings can specify any type of information or settings related to the surgical procedure, surgeon, or patient. For example, such settings may specify the type of procedure, a step of the procedure, surgical plan settings, patient parameters, the operative side of surgical object, surgical tool or manipulator settings, and the like. These settings could affect when content is presented on the exterior-facing display EFD. Sensor settings can change settings for any of the described sensors of the HMD 200, such as operating parameters, sensitivity, or toggling such sensors on/off. System settings can adjust any of the general operating settings of the HMD 200, such as memory settings, sound settings, power settings, display settings, activation settings, communication settings, system information, notification settings, or the like. These settings are provided only as an example and are not limited to the exact configuration or wording shown. Any of these settings or configurations may be utilized independently or combination to directly configured when, where and how content is displayed on the exterior-facing display EFD. Additionally, any of these settings may be pre-set default or automatically configured settings for the HMD 200, without requiring user interacting with the GUI.

The time-of-flight sensor(s) TFS of the HMD 200 can also provide input or information that can influence content presentation on the exterior-facing display EFD. For example, the time-of-flight sensor TFS can be used to determine a relative spatial relationship between the HMD 200 and the tracking system TRKSYS. This may be advantageous when the HMD 200 or tracking system TRKSYS is/are moving. The relative spatial relationship can be processed by the controller 210 to determine when or how to display the content on the exterior-facing display EFD. For instance, the relative spatial relationship may cause a change in presentation or pose of the content. The location of the content on the exterior-facing display EFD can be changed to react to a movement of the HMD 200, for example, to react to acceleration or velocity changes. The HMD 200 may also communicate time-of-fight measurements to the tracking system TRKSYS to enable the tracking system to offset latency. In other examples, the trackable graphic TG can be encoded with time stamps to facilitate synchronization with the tracking system TRKSYS. The encoding can consider measurements from the time-of-flight sensor TFS.

In another implementation, the HMD 200 can obtain information about the tracking accuracy or tracking parameters of one or more of the various tracking systems TRKSYS. The accuracy or parameters, for example, can be defined relative to a primary viewing axis of the tracking system TRKSYS. Based on this obtained information, the exterior-facing display EFD may present a fixed trackable graphic TG optimized for tracking accuracy or tracking parameters of the tracking system TRKSYS. Additionally, or alternatively, dynamic trackable graphics TG could be used in combination with time-of-flight calculations to improve the tracking accuracy in any direction. The dynamic trackable graphics TG could be turned off after the fixed trackable graphic TG is optimized for the current tracking system TRKSYS and HMD 200 positions. These techniques address the deficiency of conventional static tracking devices that exhibit degradation in tracking accuracy in response to the tracking device not directly facing the respective tracking system.

The transceiver TX of the HMD 200 can receive information from the tracking system TRKSYS that may affect when or how content is displayed on the exterior-facing display EFD. Such communication may be, for example, the tracking system TRKSYS sending a command initialize the HMD 200 and display content, such as the trackable graphic TG. Other types of communication are contemplated that may configure how the HMD 200 operates, including power settings, display settings, communication settings, authentication, sensor settings, and the like.

The proximity sensor(s) PS of the HMD 200 may influence when or how content is displayed on the exterior-facing display EFD. The proximity sensor PS can detect absence of environmental activity, and in response, place the HMD 200 or exterior-facing display EFD in a sleep mode to conserve energy. In another example, the proximity sensor PS can detect presence of environmental activity to ensure the HMD 200, exterior-facing display EFD, or displayed content remain active. As an ambient light sensor, the proximity sensor PS can active the presentation of content when the HMD 200 is moved or picked up. The proximity sensor PS may also detect a potential obstruction to the exterior-facing display EFD. In other examples, the exterior-facing display EFD could present a warning or notification based on proximity sensor PS measurements.

The inertial sensor(s) 217 of the HMD 200 may also influence the presentation of content on the exterior-facing display EFD. For example, if the pose of the HMD 200 is changed, measurements from the inertial sensor 217 may change an orientation of content presented on the exterior-facing display EFD (e.g., portrait mode to landscape mode). Inertial measurements may detect that the HMD 200 is being moved by a user and/or detect an undesired motion of the HMD 200. In turn, the content can be adjusted to account for such movement or undesired movement. In other examples, the exterior-facing display EFD could present a warning or notification regarding movement errors based on the inertial sensor 217 measurements.

The power source PWR of the HMD 200 can also affect content presentation on the exterior-facing display EFD. For example, when a threshold level of low power is detected from the power source PWR, the HMD controller 210 can dim the exterior-facing display EFD. When a threshold level of sufficient power is detected from the power source PWR, the HMD controller 210 illuminates the exterior-facing display EFD with full brightness. The exterior-facing display EFD can also present content related to power level, such as percentage of battery life left (e.g., 50% battery remaining).

### ii. Information from External Sources

Sources external to the HMD 200 can influence what, when, how and where content can be presented by the exterior-facing display EFD of the HMD 200. For example, information provided by the navigation controller 26 and camera controller 42 may include tracking data, tracked pose information of any surgical object being tracked, including the HMD 200, camera settings, spatial relationships or transforms between various objects, object geometry or tracker configurations, presence or absence of line-of-sight between the localizer and any tracked object, detection errors, tracking measurements, such as angle or distance of tracking between tracked object and localizer, the identification of objects being tracked, video stream VS data or images captured from optical sensors 40 of the navigation system, registration information, virtual boundary VB data, tool path data, surgical plan information, surgical step information, clinical application CA information (data, screens, video, images), notifications and warnings and the like. Such information can be presented on the exterior-facing display EFD and/or used to make determinations about content presented on the exterior-facing display EFD.

Information provided by the manipulator controller 54 can include any robot data, including but not limited to a pose of the manipulator 14, a kinematic model of the manipulator 14, registration or calibration data related to the manipulator 14, robotic tool path or boundary data, robotic errors, robot settings, robotic operational data, force/torque sensor inputs, joint (torque) measurements, attached end effector 22 information, end effector 22 operational information, notifications and warnings, and the like. Such information can be presented on the exterior-facing display EFD and/or used to make determinations about content presented on the exterior-facing display EFD.

Information provided by the tool controller can be any information related to any surgical tool 22 in the operating room, including hand-held tools or robotically controlled tools 22. Such information can be camera or video information if applicable (e.g., endoscope), tool settings, tool operating or control parameters, tool attachments, tool positional information, notifications, and warnings, etc. When a camera is provided, such as any camera described herein, any information captured by such cameras can be utilized. The information may be video or image data captured by any one or more cameras. The video can be a live video stream VS, e.g., of a surgical environment or target site TS. Such information can be presented on the exterior-facing display EFD and/or used to make determinations about content presented on the exterior-facing display EFD.

Any of these external sources can provide surgical information or surgical context to the HMD 200. The surgical information can include but is not limited to: information about a surgical object, an identity of a surgical object, information about a surgical procedure or step of the surgical procedure, surgical plan information, deviations from or compliance to surgical workflows, surgeon preferences, virtual objects (e.g., bone models, tool models), medical imaging data, virtual boundary information, cut or respective volume information, clinical information, patient information (age, sex, BMI, bone density), detection of tool use or operation, tool inventory, implant information or inventory, planned trajectories, planned cut planes, planned implant positions, joint measurements, planned joint biomechanics, soft tissue or ligament information, live video feeds of the surgical site, images captured at the surgical site, a tracking status of the HMD 200, an operation status of the tracking system TRKSYS, a location or the tracking system TRKSYS, the relative spatial relationship between any two objects (e.g., tool to anatomy, tool to tracking system, HMD to localizer, etc.), surgical notifications or warnings, and the like. Surgical information may also include any surgical information described from the numerous examples above this paragraph, including information from the exterior-facing display EFD, etc.

At 306, a memory or database can optionally be utilized to save or retrieve information, configurations, or settings for the HMD 200 and/or exterior-facing display EFD based on the various sources and information described above. The memory or database can be located at any suitable location such as a remote server, on the HMD 200, or accessible by any one or more of the controllers 26, 42, 54, 210. Content settings can be stored in the memory or database and these settings can be any of the settings described above defining when, how, what, where content is presented on the exterior-facing display EFD. Any input can be used to query these settings. For instance, any surgical information can be utilized to query the database for trackable graphic TG settings and retrieve the settings from the database for presentation of the trackable graphic TG on the exterior-facing display EFD. Any content settings can be retrieved such as the type or nature of the content, the respective location of the content on the one or more exterior-facing displays EFD and the specification of a condition or timing to trigger presentation of the content. At 308, given the numerous examples of information sources and information types, the operation or configuration of the HMD 200 and content displayed on the exterior-facing displays EFD can be specified. Again, such configurations can be static, dynamically changing during surgery, automatically performed, or manually inputted.

### 4. Examples of Exterior-Facing Display in Surgical Settings

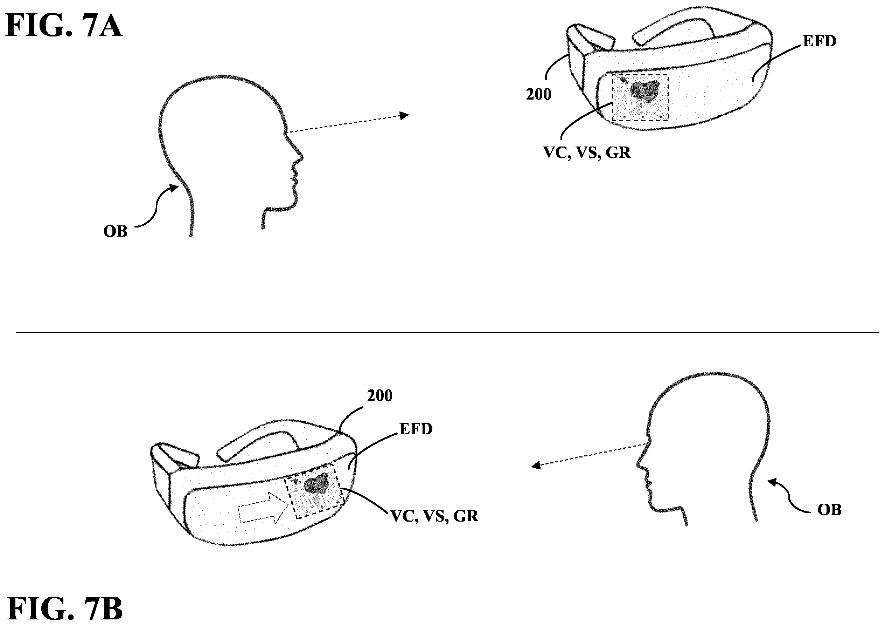

Examples of how the exterior-facing display EFD may be used in the surgical setting will now be described with reference to FIGS. 7-13. These examples are provided for illustrative purposes and are not intended to limit the scope of the disclosure. Any information presented on the display screen(s) DS in these illustrations can alternatively be presented in any manner described above and are not limited to the examples shown. These techniques can be utilized for any of the described content, including human-readable information HRI, visual/graphic content VC, or video stream VS content.

### i. Modification of Displayed Content Based on Content Visibility

Described herein are various examples by which the content displayed on the exterior-facing display EFD can change based on visibility of the content to an observer OB of the HMD 200 or tracking system TRKSYS. FIGS. 7A and 7B illustrate an example by which the content displayed by the exterior-facing display EFD changes based on the spatial relationship between the HMD 200 and an observer OB (e.g., human being in the operating room), according to one implementation. In FIG. 7A, the HMD 200 is a first pose relative to the observer OB and the HMD 200 presents visual content VC on the exterior-facing display EFD, which in this case is the video stream VS described above. Based on the techniques described above, the HMD 200 intelligently presents the visual content VC on left side of the exterior-facing display EFD because the observer OB would be able to readily detect the visual content VC at this location due to the relative poses between the HMD 200 and observer OB. Conversely, the HMD 200 does not (yet) present the visual content VC on the right side of the exterior-facing display EFD because the observer OB is not able to detect the trackable graphic TG given the relative poses between the observer OB and the HMD 200. In FIG. 7B, the relative pose between the observer OB and the HMD 200 changes such that the observer OB can no longer detect the visual content VC presented on left side of the exterior-facing display EFD. As a result, the HMD 200 dynamically moves the location of the visual content VC to the right side of the exterior-facing display EFD to enable the observer OB to view the visual content VC.

Content adjustment based on spatial relationship can be performed in numerous and ways using any of its sensors or components described herein. The HMD 200 may detect a relative pose of the HMD 200 to any one or more human observer OB and/or tracking systems TRKSYS, at discrete times or concurrently. The HMD 200 can utilize one or more of its cameras 214 to detect presence or pose of the observer/tracking system. The HMD 200 can use the time-of-flight sensor TFS to detect the distance or relative spatial relationship between the HMD 200 and the observer/tracking system. The proximity sensor PS can be used to detect presence or absence of the observer/tracking system. The transceiver TX can receive communication from the tracking system TRKSYS regarding its pose or active tracking status. These techniques can be utilized for any of the described content, including human-readable information HRI or trackable graphics TG. Any of these techniques can be used individually or in combination to determine presence/absence of, or the spatial relationship of an object near the HMD 200. Accordingly, the HMD 200 can make the intelligent determinations of when and where to display content by detecting the presence and/or pose of the HMD 200 relative to tracking systems TRKSYS or observers OB. Advantageously, such techniques can significantly optimize conveyance of information to observers in the operating room thereby avoiding human distractions. These techniques can also improve tracking accuracy and reduce disruptions during surgery resulting from tracking inaccuracies or loss of detectability of the HMD 200. Furthermore, the HMD 200 can conserve energy through such selective presentation of content.

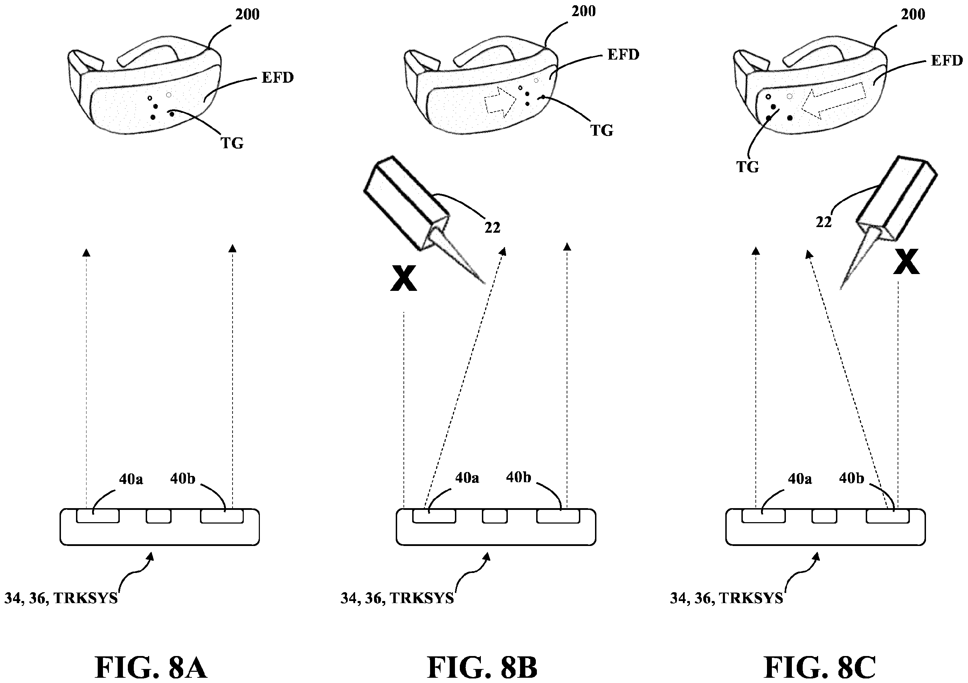

FIGS. 8A-8B illustrate a situation whereby the HMD 200 can modify content presentation on the exterior-facing display EFD based on assessment of the detectability of the HMD 200 to the observer OB/tracking system TRKSYS, which in this case is the localizer 34 of the navigation system 20. These examples describe assessing whether the camera unit 36 of the localizer 34 has a line-of-sight to the trackable graphic TG presented on the exterior-facing display EFD. However, as described, these techniques can be utilized with any displayable content and relative to any observer/tracking system TRKSYS, other than shown, such as the human observer OB in FIGS. 7A and 7B.

In FIG. 8A, the HMD 200 displays the trackable graphic TG in the center of the exterior-facing display EFD based on the spatial pose of the HMD 200 relative to the tracking system TRKSYS. The tracking system TRKSYS has clear visibility to the trackable graphic TG, without obstructions. In this example, the tracking system TRKSYS has a first sensor unit 40a and a second sensor unit 40b spaced apart from one another on the camera unit 36. Both of these sensor units 40a, 40b have line-of-sight of the trackable graphic TG.

In FIG. 8B, an object is introduced between the tracking system TRKSYS and the HMD 200. In this example, the object is a tool 22 used during surgery, such as a pointer tool. However, any object can cause such obstructions, such as any type of tool, a human being, the manipulator 14, or the like. The obstructing object is in a first pose in FIG 8B, which specifically impairs visibility of a first sensor unit 40a of the tracking system TRKSYS (shown by an "X"). The obstruction can be detected by the HMD 200 and/or the tracking system TRKSYS using any of the techniques described above, such as by the HMD 200 using its camera 214 or the tracking system TRKSYS processing signals from the optical sensors 40. In response to detection of the obstruction to the first sensor unit 40a, the one or more described controllers command movement of the trackable graphic TG to an updated pose (position and/or orientation). Namely, in FIG. 8B, the trackable graphic TG is moved, from the center to the right side of the exterior-facing display EFD. Movement of the trackable graphic TG in this manner mitigates the obstruction by placing the trackable graphic TG in an updated location that is visible to both sensor units 40a, 40b. Particularly, the first sensor unit 40a can obtain a line-of-sight to the trackable graphic TG since line-of-sight avoids the obstructing object.

In FIG. 8C, the obstructing object is moved to a second pose, which now impairs visibility of a second sensor unit 40b. In response to detection of the obstruction to the second sensor unit 40b, the one or more described controllers command movement of the trackable graphic TG from the right side to the left side of the exterior-facing display EFD. This enables the second sensor unit 40b to preserve a line-of-sight to the trackable graphic TG such that the trackable graphic TG can remain visible to both sensor units 40a, 40b. This technique can be repeatedly performed during surgery for any type of content and in response to any type of obstruction, and for any type of observer/tracking system TRKSYS. Advantageously, such techniques can significantly increase tracking accuracy and reduce disruptions during surgery resulting from tracking inaccuracies or loss of detectability of the HMD 200.

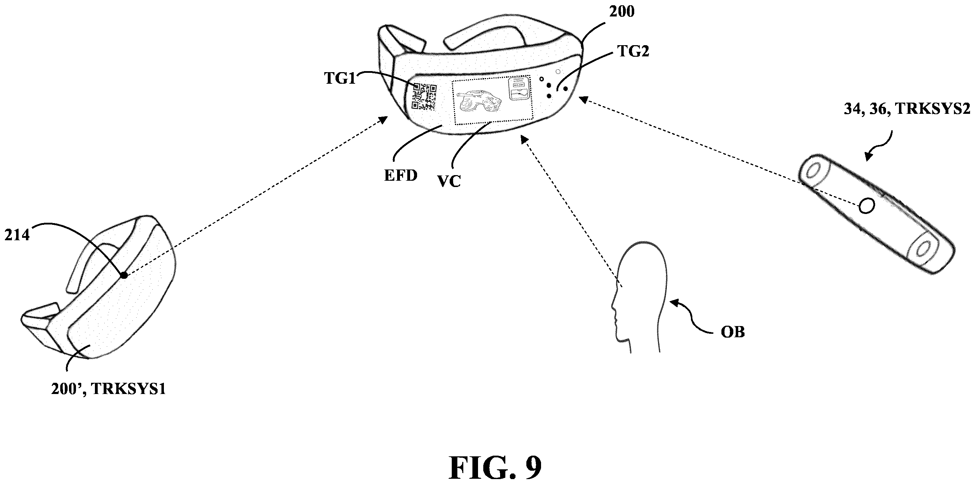

FIG. 9 illustrates the versatility of the described techniques with an example whereby the HMD 200 is utilized with multiple observers, i.e., a first tracking system TRKSYS1, a second tracking system TRKSYS2, and a human observer OB. Specifically, in this example, the first tracking system TRKSYS1 is a second HMD 200' (assumed to be worn by another person), and the second tracking system TRKSYS2 is the localizer 34/camera unit 36 of navigation system 20. The two tracking systems, TRKSYS1, TRKSYS2 and the observer OB observe the HMD 200 from three different perspectives. Using any of the sources, information, sensors, cameras, controllers, or techniques described above, the HMD 200 can receive input that define control what content to display on the exterior-facing display EFD and where to present such content on the exterior-facing display EFD based on identification and pose of the respective observer to the HMD 200. Specifically, in response to identification of the first tracking system TRKSYS1, the HMD 200 presents a first trackable graphic TG1 that is specifically customized to enable the first tracking system TRKSYS1 to track the HMD 200. For example, the first trackable graphic TG1 includes a QR code that may better be suited for detection by the camera 214 of the second HMD 200'. Based on the respective pose of the first tracking system TRKSYS1, the HMD 200 presents the first trackable graphic TG1 at a left-side of the exterior-facing display EFD to optimize visibility of the first trackable graphic TG1 to the pose of the first tracking system TRKSYS1. In response to identification of the second tracking system TRKSYS2, the HMD 200 presents a second trackable graphic TG2 that is specifically customized to enable the second tracking system TRKSYS2 to track the HMD 200. For example, the second trackable graphic TG2 can include digital fiducials that may better suited for detection by the optical sensors 40 of the localizer 34. Based on the respective pose of the second tracking system TRKSYS2, the HMD 200 presents the second trackable graphic TG2 at a right-side of the exterior-facing display EFD to optimize visibility of the second trackable graphic TG2 to the pose of the second tracking system TRKSYS2. Detection of the human observer OB causes the HMD 200 to present visual/graphic content VC in the center of the exterior-facing display EFD. The specific content presented can be customized based on any information related to the observer OB or the surgical procedure. The HMD 200 can dynamically change what is presented on the exterior-facing display EFD based on detected changes related to any of the described observers. For instance, if the first and second tracking systems TRKSYS1, TRKSYS2 were to swap poses relative to the poses shown in FIG. 9, the HMD 200 can correspondingly swap the positions of the first and second trackable graphics TG1, TG2. If the first tracking system TRKSYS1 were to no longer be present, the HMD 200 can dynamically remove presentation of the first trackable graphic TG1 and shift and expand presentation of the visual/graphic content VC from the center to the left and center of the exterior-facing display EFD. Other examples of dynamically modifying the displayed content on the exterior-facing display EFD are contemplated.

Furthermore, in addition to facilitating tracking, the HMD 200 in this example may be utilized to initially register the tracking systems TRKSYS1, TRKSYS2 to each other, or to a common coordinate system. The HMD 200 may operate as a common registration device to facilitate registration of the tracking systems TRKSYS1, TRKSYS2. By each tracking system TRKSYS1, TRKSYS2 detecting the respective trackable graphic TG1, TG2, transforms can be computed from the second HMD 200' to the localizer 34, and vice versa. Such transforms include the transform between each tracking system TRKSYS1, TRKSYS2 to the exterior-facing display EFD, and transforms based on the known relationship between the exterior-facing display EFD and each trackable graphics TG1, TG2. The transforms can be combined to enable the HMD 200 and localizer 34 to be registered to one another. Accordingly, the HMD 200 also eliminates the need for specialized registration devices. Furthermore, the HMD 200 can be utilized for various surgical purposes. In some cases, the HMD 200 can implement different operating modes, such as a tracking mode or registration mode, wherein the contents displayed on the exterior-facing display EFD are customized for the use of the HMD 200 (e.g., for tracking, navigation, or registration).

Advantageously, using these techniques one HMD 200 can utilize its exterior-facing display EFD to enable the HMD 200 to be tracked by multiple tracking systems at the same or various times. In turn, these techniques significantly reduce complexity and cost to the surgical system 10 by eliminating specialized trackers specifically configured for each tracking system. Also, the tracking accuracy is improved by dynamic presentation and movement of the trackable graphics based on the identification and relative poses of the tracking systems. Furthermore, the human observer can readily obtain relevant information directly from the HMD 200, whereas such information may otherwise not be available to the observer without having to look for such information elsewhere.

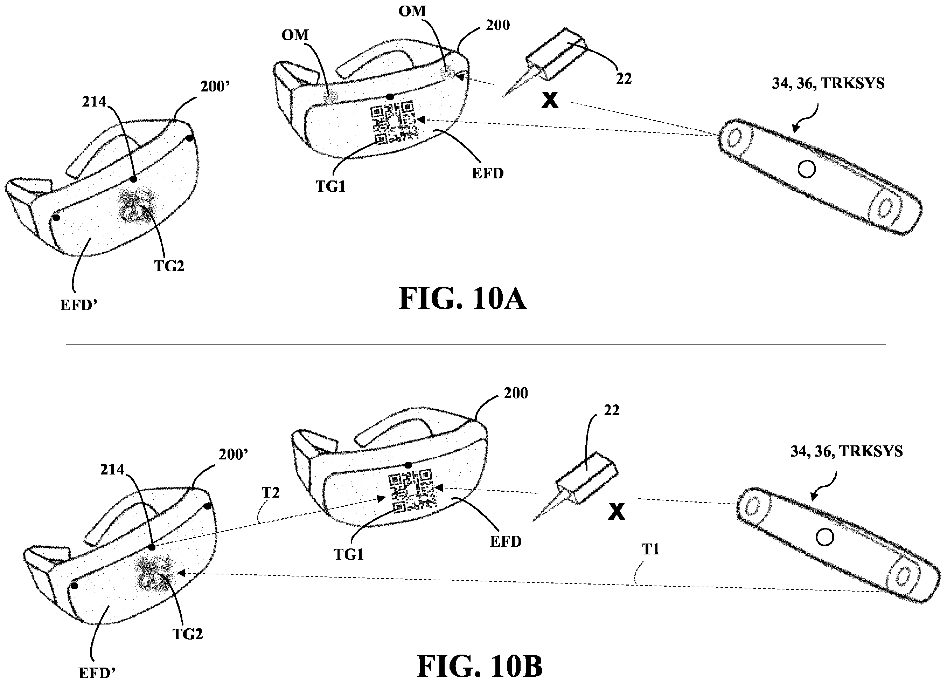

FIGS. 10A and 10B illustrates additional examples using first and second HMDs 200, 200' with a tracking system TRKSYS. These example illustrate the versatility in the described techniques dynamically accounting for loss in detectability between the tracking system TRKSYS and the first HMD 200. The first HMD 200 includes a first exterior-facing display EFD that can present a first trackable graphic TG1. The first HMD 200 also includes optical markers OM. The second HMD 200' comprises camera(s) 214 and a second exterior-facing display EFD that can present a second trackable graphic TG2. In this example, the tracking system TRKSYS is the localizer 34/camera unit 36 which can detect infrared signals from the optical markers OM and visible light signals (e.g., from the trackable graphics TG1, TG2).

In a first scenario as shown in FIG. 10A, the tracking system TRKSYS is actively tracking the first HMD 200 by detecting the optical markers OM. However, an obstruction is detected (e.g., surgical tool 22) between the tracking system TRKSYS and the first HMD 200 that prevents or obscures detection of the optical markers OM, which in turn causes a loss of tracking of the first HMD 200. In response to detection of this obstruction, the first HMD 200 immediately presents the first trackable graphic TG1 on the exterior-facing display EFD to enable the tracking system TRKSYS to maintain tracking of the first HMD 200.

In a second scenario as shown in FIG. 10B, the tracking system TRKSYS is actively tracking the first HMD 200 by detecting the first trackable graphic TG1 and actively tracking the second HMD 200' by detecting the second trackable graphic TG2. Each trackable graphic TG1, TG2 can be specifically or uniquely defined for the respective HMD 200, 200' and/or the respective user wearing the HMD 200, 200'. Here, an obstruction is detected (e.g., surgical tool 22) between the tracking system TRKSYS and the first HMD 200 that prevents or obscures detection of the first trackable graphic TG1, which in turn causes a loss of tracking of the first HMD 200. However, the second HMD 200' remains detectable by the tracking system TRKSYS and the tracking system TRKSYS can obtain a first spatial relationship (T1) between the second HMD 200' and the tracking system TRKSYS. In response to detection of this obstruction, the second HMD 200' immediately uses its camera(s) 214 to detect the first trackable graphic TG1 presented on the first HMD 200. The second HMD 200' can do so because of its unobstructed view of the first trackable graphic TG1. Throughout the duration of the detected obstruction, the second HMD 200' can communicate, to the tracking system TRKSYS, tracking data related to a second spatial relationship between the second HMD 200' and the first HMD 200 (T2). The tracking system TRKSYS can combine this tracking data and spatial relationships (T1 + T2) to maintain tracking of the first HMD 200 during the obstruction.

When multiple HMDs 200, 200' with exterior-facing displays EFD, EFD' are utilized, such as in the examples of FIGS. 10A and 10B, numerous examples are contemplated by which the exterior-facing displays EFD, EFD' may collectively present content. The HMDs 200, 200' can communicate with each other and the one or more controllers can coordinate when, what, where, and how content is presented on the various exterior-facing displays EFD, EFD'. For example, the same visual/graphic content VC can be displayed on the exterior-facing displays EFD, EFD.' Knowing that this content is presented in a duplicative manner, the one or more controllers may take advantage of this duplicative content to temporarily present other content, as needed. For instance, the first HMD 200 could immediately present a trackable graphic TG or human-readable information HRI on the first exterior-facing display EFD instead of the visual/graphic content VC. In other instances, the HMDs 200, 200' could coordinate when, where, and how to present the content depending on the relative location and/or viewing perspective of the observer OB. The first HMD 200 may present on the first exterior-facing display EFD a guidance region GR showing interactions between the virtual tool 22' and the virtual target site TS', while the second HMD 200' presents on the second exterior-facing display EFD' a sub-feature of this guidance region GR. For example, if the guidance region GR relates to anatomical registration, the second exterior-facing display EFD' could present a "tool distance to anatomy" sub-window of the guidance region GR, or a text/graphic warning or notification related to anatomical registration, e.g., "more points needed," "registration complete," etc. This coordination of the exterior-facing displays EFDs of multiple HMDs 200, 200' can be utilized for any aspect of a surgical procedure beyond the examples described. Similar techniques can be used for preoperative planning, operating room setup screen, anatomical registration, intraoperative planning, anatomical preparation, post-operative evaluation, or the like.

### ii. Customization of Displayed Content Based on Metadata

Described herein are examples involving the transmission of metadata between the HMD 200 and any suitable tracking system TRKSYS. The metadata can be used for influencing when, where, how and what content is presented on one or both of the interior-facing display IFD and/or exterior-facing display EFD of the HMD 200. Metadata can include any data or information described above and the metadata can be derived from any source describe above and therefore is not fully repeated for simplicity in description. In some instances, metadata can include HMD user identity information, surgical information, etc. In the examples below, the tracking system TRKS remotely observes and interacts with the HMD 200 and the interior-facing display IFD of the HMD 200 is enlarged for illustrative purposes.

### a. Metadata From HMD

FIG. 11 illustrates the HMD 200 and tracking system TRKSYS, as well as a method 400 or sequence diagram of events that involve generation/customization of content based on metadata. The particular steps or sequence of steps need not be limited to that shown and can vary depending on various circumstances. At 402, the HMD 200 generates a trackable graphic TG that includes, encodes, or otherwise comprises metadata. For example, the trackable graphic TG can be a dynamic QR code. At 404, the HMD 200 may optionally display content on its interior-facing display IFD. Additionally, or alternatively, at 404, the HMD 200 may display content on its exterior-facing display EFD. This content can be a virtual object VO, or any other content described herein (visual/graphic VC, human-readable information HRI, video stream VS, or the like). As shown in FIG. 11, the content presented on the interior-facing display IFD includes a virtual object VO including the described guidance region GR combined onto real-world views. The content presented on the exterior-facing display EFD includes human-readable information HRI. Again, any other type of virtual object(s) can be presented. The content presentation may not occur at the described sequence shown at 404 and can occur at other times.

At 406, the HMD 200 presents the trackable graphic TG comprising metadata on the exterior-facing display EFD. At 408, the tracking system TRKSYS detects the trackable graphic TG and obtains the metadata associated with the trackable graphic TG. The tracking system TRKSYS processes the metadata at 410 to facilitate communication back to the HMD 200. Namely, in one scenario, based on the received metadata, the tracking system TRKSYS can generate content for presentation on interior-facing display IFD and/or exterior-facing display EFD of the HMD 200. This content can be a first instance of the content, e.g., assuming the content is not currently being presented on the interior-facing display IFD and/or exterior-facing display EFD. In another scenario, based on the received metadata, the tracking system TRKSYS can customize an existing content, e.g., the content currently being presented on the interior-facing display IFD and/or exterior-facing display, at 404. For instance, the tracking system TRKSYS receives metadata defining the identity of the HMD 200 user. The tracking system TRKSYS can use the identity information to retrieve surgical information tagged to the user. The surgical information can include a surgical plan data, surgical preferences, surgical workflow or tool preferences, or the like. The content that is generated or customized can be tagged or encoded with the identity information or retrieved surgical information. For example, if the content was not currently presented at 404, the generated content can include the guidance region GR which can be created into the content based on a surgeon's workflow preferences that such a guidance region GR be presented during a specific surgical step. On the other hand, if the guidance region GR was previously presented at 404, the tracking system TRKSYS can further customize this guidance region GR by changing or adding further features to the displayed content. For instance, the customized content can include a virtual surgical guide, such as a virtual target axis, plane, or boundary or can include additional medical imaging data or bone and/or implant models. These techniques can also define where, when, how and what content are presented on the interior-facing display IFD and/or exterior-facing display according to any technique described in U.S. Provisional Patent App. No. 63/551,719, filed February 9, 2024, and entitled "Extended Reality Systems and Methods for Surgical Applications", the entire contents of which are hereby incorporated by reference.

At 412, the tracking system TRKSYS transmits the generated/customized content to the HMD 200. At 414, the HMD 200 presents the generated/customized on one or both of the interior-facing display IFD and/or the exterior-facing display EFD. The described techniques can be used to generate/customize any other type of content for the HMD 200, including text, warnings, messages, video, or any other content described herein. Accordingly, by embedding metadata in the trackable graphic TG, advanced features can be implemented by the tracking system TRKSYS that are otherwise not possible with conventional tracking techniques. For example, the content generation/customization intelligently adapts to the user of the HMD 200 to provide user-specific content at appropriate times.

### a. Metadata From Tracking System

FIG. 12 illustrates the HMD 200 and tracking system TRKSYS, as well as a method 500 or sequence diagram of events that involve generation/customization of content based on metadata. The particular steps or sequence of steps need not be limited to that shown and can vary depending on various circumstances.

At 502, the tracking system TRKSYS obtains metadata. This metadata can be derived from the tracking system TRKSYS itself, or from any other source. For example, the metadata can be based on data that the tracking system TRKSYS detects with the localizer 34. In other examples, the metadata can include HMD user identity information, surgical information, etc. Metadata can include any data or information described above and the metadata can be derived from any source describe above.

At 504, the HMD 200 optionally may already be displaying content on its interior-facing display IFD and/or the exterior-facing display EFD. This content can be a virtual object VO, or any other content described herein (visual/graphic VC, human-readable information HRI, video stream VS, or the like). As shown in FIG. 12, the content presented on the interior-facing display IFD includes a virtual object VO including the described guidance region GR combined onto real-world views. The content presented on the exterior-facing display EFD includes human-readable information HRI. Again, any other type of virtual object(s) can be presented. The content presentation may not occur at the described sequence shown at 404 and can occur at other times.

At 506, the tracking system TRKSYS transmits the metadata to the HMD 200. This transmission can involve any type of communication scheme or components described above, such as the transceiver TX of the HMD 200, a wired connection, or the like. Based on the received metadata, the HMD 200, at 508, can generate content for presentation on interior-facing display IFD and/or exterior-facing display EFD of the HMD 200. This content can be a first instance of the content, e.g., assuming the content is not currently being presented on the interior-facing display IFD and/or exterior-facing display EFD at 504. In another scenario, based on the received metadata, the HMD 200 can customize an existing content, e.g., the content currently being presented on the interior-facing display IFD and/or exterior-facing display, at 504. For instance, the HMD 200 can receive metadata defining the identity of the HMD 200 user and surgical information. The surgical information can include a surgical plan data, surgical preferences, surgical workflow or tool preferences, or the like. The content that is generated or customized by the HMD 200 can be tagged or encoded with the identity information or retrieved surgical information. For example, if the content was not currently presented at 504, the generated content can include the guidance region GR which can be created into the content based on a surgeon's workflow preferences that such a guidance region GR be presented during a specific surgical step. On the other hand, if the guidance region GR was previously presented at 504, the HMD 200 can further customize this guidance region GR by changing or adding further features to the displayed content. For instance, the customized content can include a virtual surgical guide, such as a virtual target axis, plane, or boundary or can include additional medical imaging data or bone and/or implant models. These techniques can also define where, when, how and what content are presented on the interior-facing display IFD and/or exterior-facing display according to any technique described in U.S. Provisional Patent App. No. 63/551,719, filed February 9, 2024, and entitled "Extended Reality Systems and Methods for Surgical Applications", the entire contents of which are hereby incorporated by reference.

At 510, the HMD 200 presents the generated/customized on one or both of the interior-facing display IFD and/or the exterior-facing display EFD. The described techniques can be used to generate/customize any other type of content for the HMD 200, including text, warnings, messages, video, or any other content described herein. Accordingly, by receiving metadata from the tracking system TRKSYS, advanced features can be implemented by the HMD 200 that are otherwise not possible with conventional tracking techniques. For example, the content generation/customization intelligently adapts to the user of the HMD 200 to provide user-specific content at appropriate times.

### iii. HMD Latency Techniques

FIG. 13 illustrates the HMD 200 and tracking system TRKSYS, as well as a method 600 or sequence diagram of events that involve compensation of latency in the presentation of content on the HMD 200. The particular steps or sequence of steps need not be limited to that shown and can vary depending on various circumstances.

At 602, the tracking system TRKSYS generates content that includes one or more time stamps. This content and time stamps can be derived from the tracking system TRKSYS itself, or from any other source. This content can be a virtual object VO, or any other content described herein (visual/graphic VC, human-readable information HRI, video stream VS, or the like). The content can be derived from any of the various sources described above. A clock source can be used to generate the time stamps. The clock source can be run by the tracking system TRKSYS or any described controller. The time stamps can be embedded or encoded in the content or otherwise associated therewith. At 604, the tracking system TRKSYS transmits the content with time stamps to the HMD 200. This transmission can involve any type of communication scheme or components described above, such as the transceiver TX of the HMD 200, a wired connection, or the like.

At 606, the HMD 200 may display the received content on its interior-facing display IFD and/or the exterior-facing display EFD. This content can be a virtual object VO, or any other content described herein (visual/graphic VC, human-readable information HRI, video stream VS, or the like). The content presentation may not occur at the described sequence shown at 606 and can occur at other times. As shown in FIG. 13, the content presented on the interior-facing display IFD includes a virtual object VO implemented as a virtual panel VP illustrating an example guidance region GR combined onto real-world views. The content presented on the exterior-facing display EFD includes visual content VC of the same guidance region GR. Again, any other type of content can be presented. As described above, the guidance region GR content displays real-time spatial representations between tracked objects. Contents such as the guidance region GR, or other types of content that involves time-sensitive information, may be sensitive to latency. The techniques described herein provide solutions to mitigate such latency.

At 608, based on the received content with time stamps, the HMD 200 can generate a trackable graphic TG that includes time stamps. The trackable graphic TG can be dynamically updated and synchronized with the time stamps that were received by the HMD 200 and that were provided with the content. The time stamps may correspond to or may be derived from the time stamps received from the tracking system TRKSYS. In other examples, the time stamps may be independently created by a clock source provided by the HMD controller 210. The time stamps may be embedded or encoded with the trackable graphic TG. In one example, the trackable graphic TG is a dynamic QR code.

At 610, the trackable graphic TG with time stamps is presented on the exterior-facing display EFD of the HMD 200. At 612, the tracking system TRKSYS detects the trackable graphic TG with time stamps. The time stamp generally corresponds to the moment of acquisition by the tracking system TRKSYS. This acquisition can occur at one time or at a plurality of times. Depending on the number of acquisitions of the trackable graphic TG, any number of time stamps can be detected by the tracking system TRKSYS. At 614, the tracking system TRKSYS evaluates the time stamps. The tracking system TRKSYS can compare the obtained time stamp(s) with the clock source to evaluate a latency related to presentation of the content. For example, the tracking system TRKSYS can compare the obtained time stamp(s) with the time stamp(s) provided with the content at 602 or with the clock source used to create the time stamp(s) at 602.

At 616, the tracking system TRKSYS is configured to detect, from the obtained time stamp(s), presence of the latency related to presentation of the content. In some cases, the tracking system TRKSYS can detect presence of latency in response to a time difference between the time stamp(s) being greater than a predefined threshold (e.g., 5 milliseconds, 10 milliseconds, etc.). The tracking system TRKSYS is configured to offset the latency by synchronizing or re-synchronizing the content. New or updated content can be generated based on the latency values, for example. Content that is transmitted can also include an alert or notification for presentation on the interior-facing display of the HMD 200 to inform of detected presence of the latency (as shown by the presented virtual object VO in FIG. 13). Such alerts can also be displayed on the exterior-facing display EFD. In other scenarios, the tracking system TRKSYS may not detect any latency or any latency above the threshold latency. If such scenario occurs, the tracking system TRKSYS may not need to perform any re-synchronizing or re-transmitting the content. In some cases, responsive to no latency being detected, the tracking system TRKSYS can transmit to a notification to the HMD 200 for presentation on its interior-facing display IFD and/or the exterior-facing display EFD to confirm that the content is properly synchronized. For example, as shown in FIG. 13, the exterior-facing display EFD presents human-readable information HRI, e.g., "SYNC", to indicate that the content that is presented by the HMD 200 is synchronized. Other examples of conveying such information can be like any of the techniques described above.

At 618, the tracking system TRKSYS is configured to transmit or re-transmitting the content to the controller of the HMD 200 for presentation on its interior-facing display IFD and/or the exterior-facing display EFD. At 620, the HMD 200 presents the synchronized content on one or both of the interior-facing display IFD and/or the exterior-facing display EFD. The described techniques can be used to synchronize any other type of content for the HMD 200, including text, warnings, messages, video, or any other content described herein. Accordingly, by using time stamps from the tracking system TRKSYS, advanced features can be implemented by the HMD 200 that are otherwise not possible with conventional techniques. For example, the content synchronization can be dynamically and automatically updated without requiring users to spend valuable time troubleshooting latency problems. By providing notifications, the users can be informed of such latency problems.

### 5. Example Technical Solutions and Advantages Provided by Tracker Device

The configurations and techniques involving the improved HMD 200, tracker system(s) TRKS, software or non-transitory computer readable medium(s), or methods that utilize and involve the exterior-facing display(s) EFD provide significant advantages over conventional techniques.

Firstly, the configurations and techniques described herein virtually eliminate the need to create or physically design a unique tracker for each HMD 200 that requires tracking. The HMD 200 can be tracked by any number of tracking system TRKSYS in the same setting, merely by associating the trackable graphic TG with the appropriate tracking system TRKSYS. Also, one HMD 200 can be tracked by multiple tracking systems at the same or separate times. Accordingly, the improved HMD 200 significantly reduces complexity and cost to the surgical system.

Furthermore, the HMD 200 is intelligently controllable to dynamically adjust its exterior-facing display EFD to various conditions. The exterior-facing display EFD exhibits adaptable and changing configurations to accommodate complex environmental conditions. The exterior-facing display EFD can display content or trackable graphics TG in a versatile manner, without being limited to a pre-set physical arrangement. The poses (position or orientation), shape, style, or arrangement of the content or trackable graphics TG can be actively controlled, changed, or moved on the exterior-facing display EFD of the HMD 200. One HMD 200 can coordinate what, when, how and where to present content on its exterior-facing display EFD. Multiple HMDs 200 can coordinate what, when, how and where to present content on their respective exterior-facing displays EFD. The HMD 200 can adjust such content or trackable graphics TG to offset sub-optimal placement of the HMD 200 or maintain visibility of the information or trackable graphics TG relative to the tracking system TRKSYS. Content or trackable graphics TG can be visible in 360 degrees. As such, the improved HMD 200 is less susceptible to tracking inaccuracies and losing line-of-sight to the tracking system TRKSYS seeking to track the object. Therefore, the HMD 200 can reduce interruptions and save valuable time in the surgical environment.

Moreover, by utilizing the exterior-facing display(s) EFD, the configurations and techniques described herein enable substantial new abilities to provide functionality beyond merely tracking the HMD 200. The HMD 200 can communicate surgically meaningful content (such as text, graphics, or video) to the user and such content can be provided at surgically relevant times and customized to prevent display of excessive content. Multiple HMDs 200 can virtually replace a surgical navigation system by detecting one another and communicating and coordinating the display of content with each other. Metadata can be utilized to customize the content to the HMD 200, and such customization can be specifically for the user of the HMD 200 and their preferences.

Additionally, exterior-facing display EFD can be used to provide a latency correction scheme for displayed content that is not possible with conventional systems. In turn, the content synchronization can be dynamically and automatically updated without requiring users to spend valuable time troubleshooting latency problems. By providing notifications, the users can be informed of such latency problems. The described advantages are not intended to limit the scope of the invention. Other advantages can be readily understood from the detailed description and Figures.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

## Claims

1. A surgical system (10) comprising:
a head-mounted device (HMD)(200) comprising:
a controller (210),
an interior-facing display (IFD) configured to face the eyes of a wearer of the HMD (200) and to present a virtual object (VO) in conjunction with a real-world view, and
an exterior-facing display (EFD) configured to present a trackable graphic (TG) that is computer-generated; and
a tracking system (TRKSYS) comprising a camera (34, 36, 214) being spatially separated from the HMD (200), the tracking system (TRKSYS) being configured to detect the trackable graphic (TG) with the camera (34, 36, 214) to localize the HMD (200) in a coordinate system of the camera (34, 36, 214).

2. The surgical system (10) of claim 1, wherein to present the trackable graphic (TG), the controller (210) of the HMD (200) is configured to present the trackable graphic (TG) with:
a specified pose on the exterior-facing display (EFD);
a specified shape on the exterior-facing display (EFD);
a specified graphic type on the exterior-facing display (EFD); and/or
specified display parameters on the exterior-facing display (EFD).

3. The surgical system (10) of claim 1, wherein the controller (210) of the HMD (200) is configured to dynamically change parameters of the trackable graphic (TG) on the exterior-facing display (EFD), wherein parameters of the trackable graphic (TG) include one or more of:
a pose of the trackable graphic (TG);
a shape of the trackable graphic (TG);
a graphic type of the trackable graphic (TG); and/or
display parameters of the trackable graphic (TG).

4. The surgical system (10) of claim 3, wherein the controller (210) of the HMD (200) is configured to dynamically change parameters of the trackable graphic (TG) on the exterior-facing display (EFD) in response to changes in a relative spatial pose detected between the exterior-facing display (EFD) and the camera (34, 36, 214).

5. The surgical system (10) of claim 3, wherein the controller (210) of the HMD (200) is configured to dynamically change parameters of the trackable graphic (TG) on the exterior-facing display (EFD) in response to:
detection of presence or absence of line-of-sight between the camera (34, 36, 214) of the tracking system (TRKSYS) and the HMD (200); and/or
detected movement of the HMD (200).

6. The surgical system (10) of claim 1, wherein the controller (210) of the HMD (200) is configured to encode the trackable graphic (TG) with time stamps to facilitate synchronization with the camera (34, 36, 214) of the tracking system (TRKSYS).

7. The surgical system (10) of claim 1, wherein the controller (210) of the HMD (200) is configured to:
detect, or receive input signifying, an identify or a type of the tracking system (TRKSYS); and
generate the trackable graphic (TG) based on the identity or type of tracking system (TRKSYS).

8. The surgical system (10) of claim 1, wherein the trackable graphic (TG) is a first trackable graphic (TG1) and the controller (210) of the HMD (200) is configured to present a second trackable graphic (TG2) on the exterior-facing display (EFD), wherein the second trackable graphic (TG2) is for a second tracking system (TRKSYS2) and of a different configuration than the first trackable graphic (TG1).

9. The surgical system (10) of claim 1, wherein the controller (210) of the HMD (200) is configured to implement a graphical user interface (GUI) for presentation on one or both of the interior-facing display (IFD) or the exterior-facing display (EFD), wherein the graphical user interface (GUI) is configured to enable a user to provide input to the controller (210) of the HMD (200) to modify settings or operation of the trackable graphic (TG).

10. The surgical system (10) of claim 1, wherein the tracking system (TRKSYS) is configured to transmit the virtual object (VO) to the controller (210) of the HMD (200) in response to the camera (34, 36, 214) detecting the trackable graphic (TG), and wherein the virtual object (VO) comprises surgical information related to surgery.

11. The surgical system (10) of claim 1, wherein the trackable graphic (TG) is one or more of:
a QR code;
a dynamic QR code; and
a geometric arrangement of at least three digital fiducials.

12. The surgical system (10) of claim 1, wherein the exterior-facing display (EFD) is further configured to present one or more of:
human-readable information (HRI);
surgical graphics or imagery;
surgical video; and
a video stream (VS) obtained by the camera (34, 36, 214) of the tracking system (TRKSYS).

13. The surgical system (10) of claim 1, wherein the tracking system (TRKSYS) comprises one or more of:
another HMD (200'),
a surgical navigation system (20) including a localizer (34), and/or
a tool (22) comprising a camera.

14. A head-mounted device (HMD)(200) for use with a tracking system (TRKSYS) that includes a camera (34, 36, 214) and is spatially separated from the HMD (200), the HMD (200) comprising:
a controller (210);
an interior-facing display (IFD) configured to face the eyes of a wearer of the HMD (200); and
an exterior-facing display (EFD);
wherein the controller (210) is configured to:
obtain or generate a trackable graphic (TG) that is computer-generated; and
present the trackable graphic (TG) on the exterior-facing display (EFD) for detection by the camera (34, 36, 214) of the tracking system (TRKSYS).

15. A method of operating a surgical system (10) including a head-mounted device (HMD)(200) with a controller (210), an interior-facing display (IFD) configured to face the eyes of a wearer of the HMD (200), and an exterior-facing display (EFD); and a tracking system (TRKSYS) including a camera (34, 36, 214) being spatially separated from the HMD (200), the method comprising:
obtaining or generating, with the controller (210) of the HMD (200), a trackable graphic (TG) that is computer-generated;
presenting, with the controller (210) of the HMD (200), the trackable graphic (TG) on the exterior-facing display (EFD);
detecting the trackable graphic (TG) with the camera (34, 36, 214) of the tracking system (TRKSYS); and
localizing, with the tracking system (TRKSYS), the HMD (200) in a coordinate system of the camera (34, 36, 214) in response to detecting the trackable graphic (TG).
